# EUROPEAN PATENT APPLICATION

(11) **EP 1 746 092 A1**
(43) Date of publication of application: **24.01.2007**
(21) Application number: 05291576.6
(22) Date of filing: 22.07.2005
(51) Int. Cl.: C07D 401/12, C07D 215/36, A61K 31/4709, A61K 31/47, A61P 25/28

(54) **Compounds and methods for treatment of amyloid-B-peptide related disorders**

(71) Applicant: Exonhit Therapeutics SA, 75017 Paris (FR)
(72) Inventor: Leblond, Bertrand, 76000 Rouen (FR); Beausoleil, Eric, 75015 Paris (FR); Taverne, Thierry, 62280 Saint Martin Boulogne sur mer (FR); Desire, Laurent, 75014 Paris (FR); Schweighoffer, Fabien, F-94120 Fontenay sous Bois (FR)
(74) Representative: Tezier Herman, Béatrice

(57) **Abstract**

The present invention relates to compounds, preparation and uses thereof, particularly in the pharmaceutical industry. The present invention discloses novel compounds of the following formula (I) 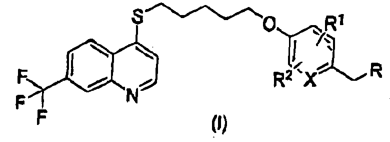 more particularly useful for the treatment of Alzheimer's disease and other similar diseases, and more specifically the inventive compounds modulate (in particular, inhibit) the level of amyloid-β peptide (Aβ) exhibited by cells or tissues; Aβ peptide is a major component of the amyloid plaques found in the brains of Alzheimer's sufferers. This invention also relates to the use of these inhibitors to prevent, treat or ameliorate the symptoms of Alzheimer's disease or any Amyloid-β-Peptide Related Disorder.

## Description

### FIELD OF THE INVENTION

The present invention relates to compounds, preparation and uses thereof, particularly in the pharmaceutical industry. The present invention discloses novel compounds more particularly useful for the treatment of Alzheimer's disease and other similar diseases, and more specifically the inventive compounds modulate (in particular, inhibit) the level of amyloid-β peptide (Aβ) exhibited by cells or tissues ; Aβ peptide is a major component of the amyloid plaques found in the brains of Alzheimer's sufferers. This invention also relates the use of these inhibitors to prevent, treat or ameliorate the symptoms of Alzheimer's disease or any Amyloid-β-Peptide Related Disorder.

### BACKGROUND OF THE INVENTION

Alzheimer's disease (AD), the leading cause of dementia in elderly persons, is a devastative degenerative disorder of the brain with important formation of amyloid plaques, neurofibrillary tangles, gliosis and neuronal loss (Hardy et al. Nat. Neurosci 1:355-358 (1998); Selkoe, D.J. In: Alzheimer disease, Ed 2 (Terry, R.D., Katzman, R., Bick, K.L., Sisodia, S.S., eds), pp 293-310. Philadelphia: Lippincott Williams and Wilkins. (1999)). There is no effective palliative or preventive treatment for the neurodegeneration. The most affected regions are cortex, hippocampus, subiculum, hippocampal gyrus, and amygdala.
The AD brain presents two distinct pathologies; 1) neurofibrillary tangles (NFT), comprised mostly of tau and 2) amyloid plaques, comprised primarily of highly hydrophobic amyloid precursor protein peptides called Aβ peptides. The characteristic Alzheimer's NFTs contain abnormal filaments bundled together in neurons and occupying much of the perinuclear cytoplasm. These filaments contain the microtubule-associated protein tau in a hyperphosphorylated form. In AD, the dysregulation of intracellular pathways responsible for amyloid precursor protein (APP) proteolytic processing results in enhanced formation of Aβ 1-42, a form of the Aβ peptide which is particularly amyloidogenic, and which appears to be central to the pathophysiology of AD (Selkoe, Neuron 32: 177-180 (2001)). Other neuropathological features include granulovascular changes, neuronal loss, gliosis and the variable presence of Lewy bodies.

The Aβ peptide is a hydrophobic peptide comprising up to 43 amino acids. Aβ has been shown to be toxic to neurons either intracellulary and extracellularly, and mediates its effects by the induction of reactive oxygen species (ROS), induction of altered gene transcription, increased susceptibility to excitotoxicity, and other processes commonly associated with neurodegenerative conditions (Ramsden et al., J. Neurochem. 79: 699-712 (2001); Shukla et al., J. Cell. Path. 5: 241-249 (2002); Green and Peers, Neurochem. 77: 953-956 (2001); Kowall et al., Neurobiol. Aging 13: 537-542 (1992); MacManus et al., J. Biol. Chem. 275: 4713-4718 (2000)). Mutations in APP, Presenilin 1 and 2 (PS1 and PS2, respectively) greatly alter APP processing, resulting in enhanced Aβ 1-42 formation. Amyloid plaques are also detected in aged patients with Down's Syndrome who survive up to the age of 30. The observed up-regulation of APP expression in Down's Syndrome is probably a cause of the development of AD in Down's patients (Rumble et al., N. Engl. J. Med. 320:1446-52 (1989); Mann, Neurobiol. Aging 10: 397-399 (1989)). Amyloid plaques are also present in the normal aging brain, although at a lower number (Vickers et al., Exp. Neurol. 141:1-11 (1996)).

Examples of disease-linked mutations include a double mutation at APP codons 670 and 671 found in a Swedish kindred, resulting in a substitution of the normal lysine-methionine dipeptide by asparagine-leucine (Mullan, et al., (1992) Nature Genetics 1:345-347). APP with K670N-M671L is reported to be associated with increased Aβ 1-40 secretion (Citron et al. (1992) Nature 360:672-674; Cai et al. (1993) Science 259:514-516), while enhanced Aβ 1-42 production is reported for APP with the V717I mutation (Cai et al. (1993), Science 259:514-516; Suzuki et al. (1994) Science 264:1335-1340). A number of other pathogenic mutations have been found within APP which are associated with hereditary forms of AD, several of which are located within the Aβ sequences. These mutations result in a phenotype different from AD, with massive amyloid accumulation in cerebral blood vessel walls. Two mutations, namely the Dutch (Glu22Gln) and the Flemish (Ala21 Gly) mutations, have been reported (Levy, et al., Science 248, 1124-1126 (1990)), (van Broeckhoven et al. (1990)), (Hendriks, et al., Nature Genet 1, 218-221 (1992)). Patients having these mutations suffer from cerebral hemorrhage and vascular symptoms. The vascular symptoms are caused by aggregation of Aβ in blood vessel walls (amyloid angiopathy). A third pathogenic intra-Aβ mutation was also discovered in an Italian family (Glu22Lys), with clinical findings similar to the Dutch patients (Tagliavini, et al., Alz Report 2, S28 (1999)). Another pathogenic AD mutation within APP, named the "Arctic mutation" (Glu22Gly), is also located within the Aβ peptide domain of the APP gene. Carriers of this mutation develop progressive dementia with clinical features typical of AD without symptoms of cerebrovascular disease. Finally, carriers of the "Iowa" mutation, carrying a Asp23Asn mutation within Aβ exhibit severe cerebral amyloid angiopathy, widespread neurofibrillary tangles, and unusually extensive distribution of Aβ 40 in plaques. (Grabowski et al., Ann. Neurol. 49: 691-693 (2001)).

Three abundant forms of APP have been identified, APP695 described by Kang et al. (1987) Nature 325: 733-736 which is designated as the "normal" APP; the 751 amino acid polypeptide (APP751) described by Tanzi et al. (1988) Nature 331: 528-530; and the 770 amino acid polypeptide (APP770) described by Kitaguchi et. al. (1988) Nature 331: 530-532. These forms arise from a single precursor RNA by alternative splicing generating the different forms of human APP ranging in size from 695-770 amino acids, which localize to the cell surface, and have a single C-terminal transmembrane domain. The Aβ peptide, which is common to each of the three splice variants of APP, is derived from a region of APP adjacent to and containing a portion of the transmembrane domain.

Three different proteases process APP *in vivo* (Vassar and Citron, Neuron 27: 419-422 (2000)). The non amyloidogenic pathway of APP processing involves the metalloproteases ADAM10, ADAM17 and possibly ADAM9 as alpha-secretases, which cleave APP 12 amino acid residues from the lumenal surface of the plasma membrane, within the Aβ domain. Hence, this pathway is not involved in Aβ production but, rather in the production of the sAPPalpha fragment which has been suggested to be neuroprotective. The first step of Aβ generation is performed by cleavage of APP by β-secretase (BACE), a type I membrane-bound aspartyl protease. BACE cleavage generates a 100-kDa soluble form (sAPP) of the ectodomain - the portion of APP that projects from the cell surface - and a 12-kDa membrane-associated intermediate peptide of 99 amino acids (termed C99) containing the N-terminus of the Aβ peptide (Vassar et al., Science 286(5440):735-41(1999). The C99 peptide is then processed by the protease gamma-secretase to yield various Aβ peptides differing in size or terminal modification (40-42 and 43 amino acid residues being the most frequent peptides found *in vivo)* (for review, see Selkoe et al., Nature 399(6738 Suppl):A23-31 (1999); Tekirian, J. Alzheimers. Dis. 3(2):241-248. (2001)). Amino acid sequence changes in APP that result in increased APP cleavage by BACE increase the likelihood of the development of Alzheimer's (Citron et al., Nature 360(6405): 672-674 (1992)). Experimental evidence suggests that APP processing is sequential and that cleavage of APP by beta-secretase is a prerequisite for gamma-secretase-mediated APP processing. Cleavage within the transmembrane region of APP by gamma-secretase results in the 40-42 residues Aβ peptide, whose elevated production and accumulation in the brain are the central events in the pathogenesis of Alzheimer's disease (Selkoe, Nature 399:23-31 (1999)). In addition, it is now clear that BACE can again cut Aβ peptide 40-42 after gamma-secretase to generate a neurotoxic Aβ34 peptide, at the expense of Aβ 40-42 (Fluhrer et al., J. Biol. Chem. 278(8): 5531-5538 (2003)). Gamma-cleavage liberates small peptides that are derived from the transmembrane region of APP and adjacent extracellular sequences. These peptides include Aβ 40 and Aβ 42, the major components of amyloid fibrils in Alzheimer's disease (Lu, D. C., et al. (2000) Nat. Med. 6, 397-404; Leissring, M. A., et al. (2002) Proc. Natl. Acad. Sci. USA 99, 4697-4702; reviewed in Selkoe, D. J. (1998) Trends Cell Biol. 8, 447-453; Haass, C., and De Strooper, B. (1999) Science 286, 916-919). In addition, gamma-cleavage generates an intracellular cytoplasmic fragment referred to as amyloid precursor protein intracellular domain ("AICD") (Kimberly, W. T., et al. (2001) J. Biol. Chem. 276, 40288-40292; Cupers, P., et al. (2001) J. Neurochem. 78, 1168-1178) that may function as a transcriptional activator (Cao, X., and Sudhof, T. C. (2001) Science 293, 115-120; Gao, Y., and Pimplikar, S. W. (2001) Proc. Natl. Acad. Sci. USA 98, 14979-14984) and have other signaling roles (Lu, D. C., et al. (2000) Nat. Med. 6, 397-404; Leissring, M. A., et al. (2002) Proc. Natl. Acad. Sci. USA 99, 4697-4702). It is believed that gamma-secretase itself depends for its activity on the presence of presenilin-1. In a manner that is not fully understood, presenilin-1 also appears to undergo autocleavage.

Many of the existing therapeutic strategies for AD have focused on β-secretase inhibition mainly because genetic knock-out of BACE have shown that cleavage et the BACE site is the rate-limiting step in Aβ production and that the lack of BACE activity is not inevitably fatal in utero (Sinha, S. & Lieberburg, Proc Natl Acad Sci U S A. 96(20):11049-53 (1999); Vassar, Adv. Drug Deliv. Rev. 54(12):1589-1602 (2002)). However, it now appears that such strategies may not be sufficient to treat or prevent AD. Due to its large catalytic pocket, BACE may prove to be a difficult target for Alzheimer's disease drug design. The enzyme is highly expressed in the pancreas, where its function may be to produce peptide hormones or other secreted proteins. And its function in the brain may similarly be to produce neuropeptides important in CNS function. Aβ deposition occurs early in the disease process, but Alzheimer's disease symptoms appear late, when it may be too late for BACE inhibitors to have much effect. Potential BACE inhibitors may also inhibit the closely related BACE2 enzyme, and its functions may also be crucial to normal physiological functions. The other likely target for Aβ lowering strategies is the gamma-secretase complex, the inhibition of which also prevents Aβ production *in vitro* and *in vivo.* Presenilins are part of the gamma-secretase complex and are required for intramembrane proteolysis of APP to yield amyloid-beta protein (De Strooper et al., Nature, 391: 387-90,1998; Steiner and Haass. Nat. Rev.Mol. Cell. Biol., 1: 217-24,2000) but are also involved in the processing of other type-I membrane proteins, including Notch or its ligands Jagged2 and Delta, ErbB4, CD44, low density lipoprotein receptor-related protein, N- and E-cadherins, nectin-1, deleted in colorectal cancer (DCC), p75 neurotrophin receptor and syndecan 3 (De Strooper and Annaert, Nat. Cell Biol., 3: E221-25,2001; Sisodia and George-Hyslop, Nat. Rev.Neurosci., 3: 281-90,2002). In particular, current gamma-secretase inhibitors present the disadvantage of inhibiting Notch signaling which results in clear toxicity in adult animals (Wong G. et al. (2004) J. Biol. Chem. 279, 12876-12882). In addition one possible disadvantage of this strategy is the possibility that the resultant increase of C83 and C99 can be problematic (learning is impaired in transgenic Tg13592 mice expressing C99).

Therefore, compositions that modulate the activity of these proteases, or the likelihood of APP to be cut, are desired instead of direct inhibitor compounds. For example, WO03057165 patent application states that the Abl inhibitor Gleevec acting as a selective competitor of ATP reduces Aβ levels, in particular levels of secreted Aβ 42 derived from the cleavage of APP, by modulating gamma-secretase activity. Since there is good evidence that high Aβ 42 levels are a major risk factor for AD, such drug may be useful in preventing, delaying or reversing the progression of AD. In another example, U.S. Patent Application 20020128319 Al states that certain non-steroidal anti-inflammatory drugs (NSAIDS) lower production and/or levels of Aβ 42 in cell cultures and modulate gamma-secretase activities. The drawback of the use of such drugs, however, is that large doses of NSAIDS are required for significant lowering of Aβ 42, and that serious gastrointestinal side effects are associated with prolonged use of NSAIDS at high doses (Langman et al., 1994, Lancet 343: 1075-1078). Also, the mechanism itself is questionable as other NSAIDs and the isoprenoid pathway in contrast enhance Aβ 42 levels (Kukar T et al., Nat Med. 11(5):545-50 (2005)) and even determine the intracellular accumulation of Aβ 42, which is the basis of its early neurotoxicity in AD (Cole S et al., J. Biol. Chem. 280(19):18755-70 (2005)). In addition, the vast majority of the current gamma-secretase modulators do not affect (i.e. do not increase or decrease) Aβ 40 levels, leaving a significant amount of this pathologic peptides unchanged.

At present, there are no effective treatments for halting, preventing or reversing the progression of Alzheimer's disease. Therefore, there is a considerable need for pharmaceutical agents capable of preventing the disease or at least slowing its progression. Compounds that are effective inhibitors or modulators of beta-secretase, that are effective inhibitors or modulators of gamma-secretase, that inhibit cleavage of APP, that are effective inhibitors of Aβ 40 and Aβ 42 production, and/or are effective at reducing amyloid beta deposits or plaques, are needed for the treatment and prevention of diseases characterized by amyloid beta deposits or plaques, such as AD.

### SUMMARY OF THE INVENTION

The invention provides compounds having valuable properties, in particular those which can be used for the preparation of medicaments. It has been found that the compounds of formula I and any pharmaceutically acceptable derivative thereof, modulate the processing of APP, reducing or arresting the production of amyloid beta peptide Aβ 40, thus preventing the formation of insoluble plaques. The inventive compounds have valuable pharmacological properties and are particularly useful in the treatment or prevention of any Amyloid-β-Peptide Related Disorder, such as AD. More specifically, the present compounds may be of particular interest in the treatment of a patient who has, or in preventing a patient from developing, a disease or condition of AD, by preventing or delaying the onset of Alzheimer's disease, slowing the progression of Alzheimer's disease, treating patients with mild cognitive impairment (MCI) and preventing or delaying the onset of Alzheimer's disease in those who would progress from MCI to AD, treating Down's syndrome, treating humans who have Hereditary Cerebral Hemorrhage with Amyloidosis of the Dutch-Type, treating cerebral amyloid angiopathy and preventing its potential consequences, i.e. single and recurrent lobar hemorrhages, or by treating other degenerative dementias, including dementias of mixed vascular and degenerative origin, dementia associated with Parkinson's disease, dementia associated with progressive supranuclear palsy, dementia associated with cortical basal degeneration, or diffuse Lewy body type of Alzheimer's disease. The invention also relates to a method for the treatment of any Amyloid-β-Peptide Related Disorder, such as AD, to a subject in need of such treatment which comprises administration of a therapeutically effective amount of a compound of formula I.

Accordingly, one object of the invention is to provide a compound having a general formula (I): 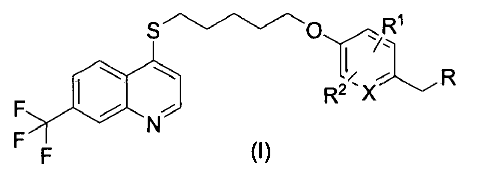 wherein:
X is CH or N;
R¹ and R², which are the same or different, represent an hydrogen atom, a halogen atom, C₁-C₆ alkyl, C₁-C₆ alkoxy group, NO₂, hydroxyl group, or NR'R", wherein R' and R", which are the same or different, represent an hydrogen atom or a C₁-C₆ alkyl group;
R is an hydrogen atom, hydroxyl, 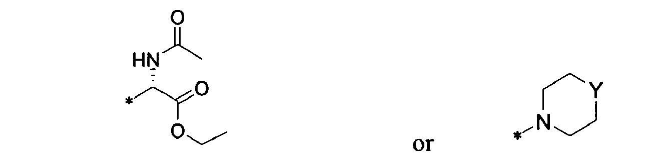
wherein Y represents an oxygen atom, a CH₂ radical or NR³, in which R³ represents an hydrogen atom, -(C=O)C(CH₃)₃, -(C=O)N(C₂H₅)₂ or -(C=O)N[CH(CH₃)₂]₂; a salt, hydrate or mixture therof.

The compounds of the present invention also include tautomers, optical and geometrical isomers and mixtures thereof. The compounds of the present invention may indeed have one or more asymmetric centers and it is intended that stereoisomers (optical isomers), as separated, pure or partially purified stereoisomers or racemic mixtures thereof are included in the scope of the invention.

The present invention also relates to pharmaceutical compositions comprising at least one compound as defined above in a pharmaceutically acceptable support, optionally in association with another active agent.

The pharmaceutical composition is more particularly intended to treat or prevent any Amyloid-β-Peptide related disorder. The Amyloid-β-Peptide related disorders include Alzheimer's disease, mild cognitive impairment, Down's syndrome, Hereditary Cerebral Hemorrhage with Amyloidosis of the Dutch-Type, cerebral amyloid angiopathy and consequences thereof, i.e. single and recurrent lobar hemorrhages, other degenerative dementias, including dementias of mixed vascular and degenerative origin, dementia associated with Parkinson's disease, dementia associated with progressive supranuclear palsy, dementia associated with cortical basal degeneration, or diffuse Lewy body type of Alzheimer's disease.

The present invention also includes methods of treating diseases or conditions as described above, comprising the administration to a subject in need thereof of an effective amount of a compound as defined above.

### DETAILED DESCRIPTION OF THE INVENTION

Within the context of the present application, the terms alkyl and alkoxy denote linear or branched saturated groups containing from 1 to 6 carbon atoms. An alkoxy group denotes an -O-alkyl group.

The alkyl groups may be linear or branched. Examples of alkyl groups having from 1 to 6 carbon atoms inclusive are methyl, ethyl, propyl, isopropyl, t-butyl, n-butyl, pentyl, hexyl, 2-methylbutyl, 2-methylpentyl, and the other isomeric forms thereof. Preferably, the alkyl groups have from 1 to 4 carbon atoms.

Halogen atom includes preferably chlorine, bromine or fluorine.

According to a particular aspect of the invention, the compounds present a formula I wherein X = CH and R is OH,

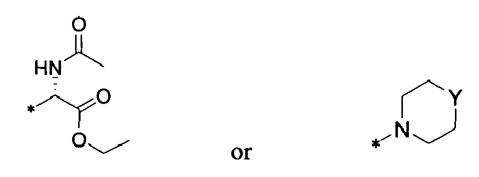

According to this embodiment, wherein X = CH and R is OH or 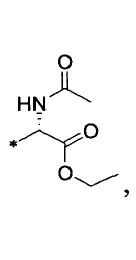 R¹ and R² both represent preferably an hydrogen atom.

According to this embodiment, wherein X = CH and R is 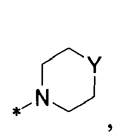 preferably Y is CH₂ and R¹ and R² are (i) both hydrogen atoms or (ii) one is hydrogen and the other is alkyl, preferably methyl (even more preferably 2-Me), halogen atom, preferably chlorine (even more preferably 2-Cl), nitro (preferably 2-NO₂), NR'R" as defined above, preferably NH₂ (even more preferably 2-NH₂), alkoxy, preferably methoxy (even more preferably 2-OMe), or (iii) both are different from hydrogen, in particular one is alkoxy, preferably methoxy (even more preferably 2-OMe) and the other is nitro (preferably 5-NO₂).

According to this embodiment, wherein X = CH and R is 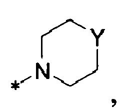 preferably Y is oxygen atom and R¹ and R² are (i) both hydrogen atoms or (ii) one is hydrogen and the other is alkoxy, preferably methoxy (even more preferably 2-OMe).

According to this embodiment, wherein X = CH and R is 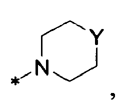 preferably Y is NR³ , with R³ as defined above, and R¹ and R² both represent H.

According to another particular aspect of the invention, the compounds present a formula I wherein X = N and R, R¹ and R² are H.

Specific examples of compounds of formula (I) which fall within the scope of the present invention include the following compounds:
4-(5-(4-((piperidin-1-yl)methyl)phenoxy)pentylthio)-7-(trifluoromethyl)quinoline dihydrochloride,
4-(5-(2-methoxy-4-((piperidin-1-yl)methyl)phenoxy)pentylthio)-7-(trifluoromethyl)quinoline dihydrochloride,
4-(5-(4-(morpholinomethyl)phenoxy)pentylthio)-7-(trifluoromethyl)quinoline dihydrochloride,
4-(5-(2-methoxy-4-(morpholinomethyl)phenoxy)pentylthio)-7-(trifluoromethyl)quinoline dihydrochloride,
4-(5-(4-((piperazin-1-yl)methyl)phenoxy)pentylthio)-7-(trifluoromethyl)quinoline trihydrochloride,
4-(5-(2-chloro-4-((piperidin-1-yl)methyl)phenoxy)pentylthio)-7-(trifluoromethyl)quinoline dihydrochloride,
4-(5-(2-nitro-4-((piperidin-1-yl)methyl)phenoxy)pentylthio)-7-(trifluoromethyl)quinoline dihydrochloride,
4-(4-(5-(7-(trifluoromethyl)quinolin-4-ylthio)pentyloxy)benzyl)-*N*,*N-*diethylpiperazine-1-carboxamide dihydrochloride,
1-(4-(4-(5-(7-(trifluoromethyl)quinolin-4-ylthio)pentyloxy)benzyl)piperazin-1-yl)-2,2-dimethylpropan-1-one dihydrochloride,
4-(4-(5-(7-(trifluoromethyl)quinolin-4-ylthio)pentyloxy)benzyl)-*N*,*N-*diisopropylpiperazine-1-carboxamide dihydrochloride,
(4-(5-(7-(trifluoromethyl)quinolin-4-ylthio)pentyloxy)phenyl)methanol hydrochloride,
2-(5-(7-(trifluoromethyl)quinolin-4-ylthio)pentyloxy)-5-((piperidin-1-yl)methyl)benzenamine trihydrochloride,
4-(5-(2-methyl-4-((piperidin-1-yl)methyl)phenoxy)pentylthio)-7-(trifluoromethyl)quinoline dihydrochloride,
(*S*)-ethyl 3-(4-(5-(7-(trifluoromethyl)quinolin-4-ylthio)pentyloxy)phenyl)-2-acetamidopropanoate hydrochloride,
4-(5-(6-methylpyridin-3-yloxy)pentylthio)-7-(trifluoromethyl)quinoline dihydrochloride and 4-(5-(2-Methoxy-6-nitro-4-((piperidin-1-yl)methyl)phenoxy)pentylthio)-7-(trifluoromethyl)quinoline.

When the compounds according to the invention are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci. 1977, 66, 2, which is incorporated herein by reference. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

The pharmaceutically acceptable salts are prepared by reacting the compound of formula I with 1 to 4 equivalents of a base such as sodium hydroxide, sodium methoxide, sodium hydride, potassium t-butoxide, calcium hydroxide, magnesium hydroxide and the like, in solvents like ether, THF, methanol, t-butanol, dioxane, isopropanol, ethanol, etc. Mixture of solvents may be used. Organic bases like lysine, arginine, diethanolamine, choline, guanidine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, fonic acid, acetic acid, citric acid, maleic acid, salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane, etc. Mixture of solvents may also be used.

The compounds according to the present invention may be prepared by various methods known to those skilled in the art. More preferably, the following convergent chemical route has been carried out.

4-(5-bromopentylthio)-7-(trifluoromethyl)quinoline was obtained from a phase transfer alkylation reaction at room temperature of 7-trifluoromethylquinolin-4-thiol by 1,5-dibromopentane using *n*-tetra-butylammonium bromide as phase transfer agent and a mixture of water:chloroform as solvent. Reductive amination (Borch, R. F. and Durst, H. D., J. Am. Chem. Soc., 91, 3996, 1969) or Leuckart-Wallach reaction (Wallach O., Ann. 272, 99, 1892) of benzaldehyde or substituted benzaldehydes with secondary amines such piperidine, morpholine or *tert*-butyl piperazine-1-carboxylate led to the corresponding 4-((amino)methyl)phenols. Those phenols were alkylated by 4-(5-bromopentylthio)-7-(trifluoromethyl)quinoline in presence of cesium carbonate in *N,N*-dimethylformamide at 25-120 °C preferably at 50-90 °C to give the free base of compounds of the invention (compound **16),** their dihydrochloride salts were obtained using a solution of HCl in MeOH (compounds **1, 5-11**). In the case of Y = NH, the trihydrochoride salt **1** was treated with triethylamine and pivaloyl chloride or a dialkylcarbamic chloride in dichloromethane at room temperature to led, after HCl treatment, to the corresponding *N*-pivaloyl amide dihydrochloride salt **3** and *N*-dialkyl ureas dihydrochloride salts **2** and **4.**

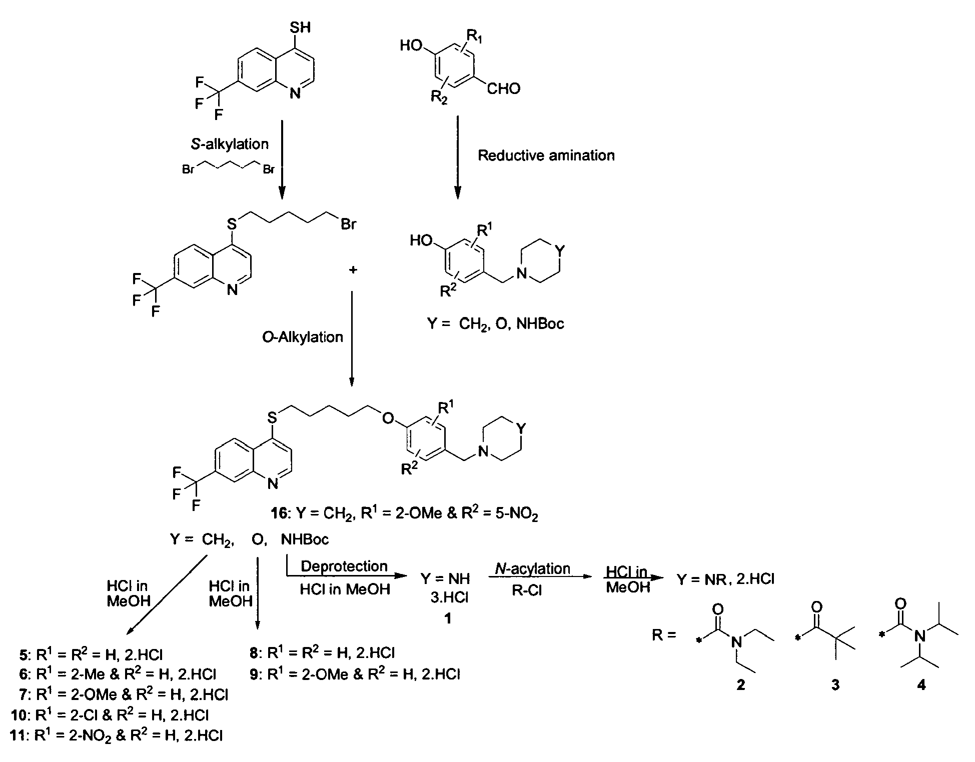

### Preparation of compounds 12 and 13 of the present invention was done as depicted in the scheme below:

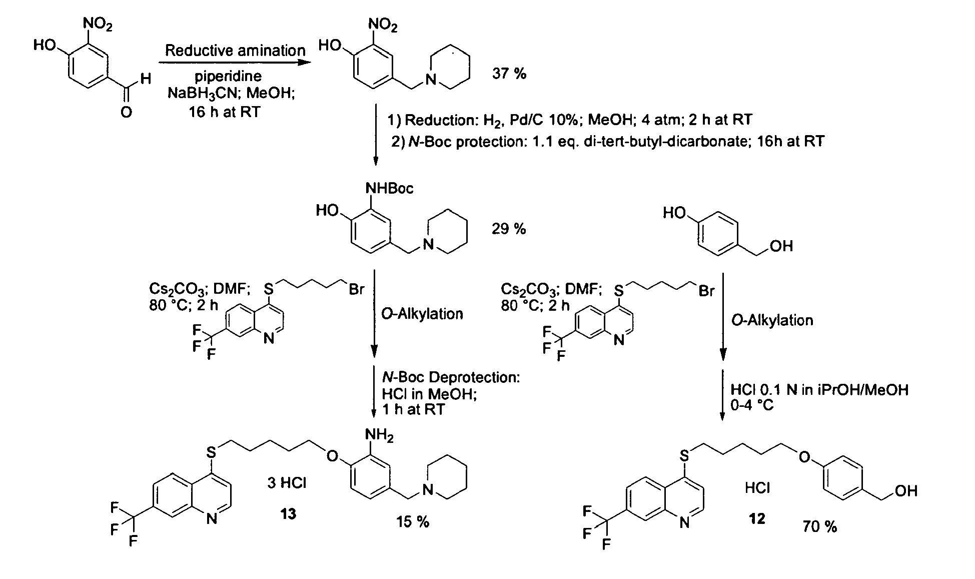

For preparation of compound **15,** 6-methylpyridin-3-ol was alkylated by 4-(5-bromopentylthio)-7-(trifluoromethyl)quinoline in presence of cesium carbonate in *N,N-*dimethylformamide and a catalytic amount of sodium iodide at 25-120 °C preferably at 90 °C to give a free base which was treated by a solution HCl in MeOH to led to compound **15** as a dihydrochloride salt.

### Preparation of compound 14 from (S)-ethyl 2-acetamido-3-(4-hydroxyphenyl)propanoate was done via a similar alkylation reaction than to obtain compound 15 excepted that no catalytic amount of sodium iodide was used.

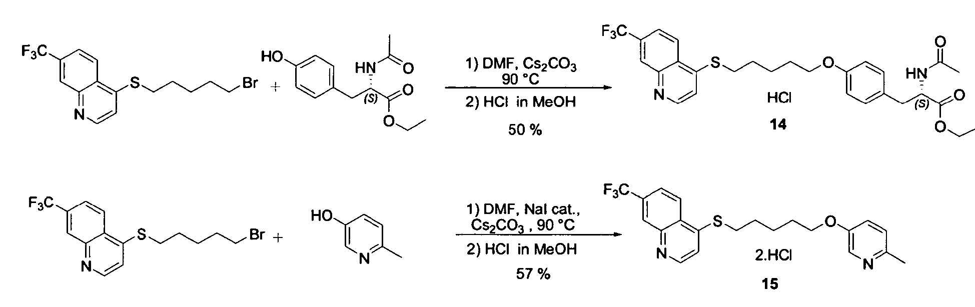

It should be understood that other ways of producing these compounds may be designed by the skilled person, based on common general knowledge and following guidance contained in this application.

As indicated above, a further object of this invention relates to a pharmaceutical composition comprising at least one compound of formula (I), as defined above, and a pharmaceutically acceptable vehicle or support. In particular, said pharmaceutical composition may be useful for preventing, treating or ameliorating the symptoms of Alzheimer's disease or any Amyloid-β-Peptide Related Disorder.

The dosages and dosage regimen in which the compounds of formula (I) are administered will vary according to the dosage form, mode of administration, the condition being treated and particulars of the patient being treated. Accordingly, optimal therapeutic concentrations will be best determined at the time and place through routine experimentation.

The compounds according to the invention can be used enterally or parenterally. Orally, the compounds according to the invention are suitably administered in the amount from about 0.1 mg per day to 1,000 mg per day. For parenteral, sublingual, intranasal, or intrathecal administration, the compounds according to the invention are suitably used in the amount from about 0.5 to about 100 mg/day; for depo administration and implants from about 0.5 mg/day to about 50 mg/day; for topical administration from about 0.5 mg/day to about 200 mg/day; for rectal administration from about 0.5 mg to about 500 mg. In a preferred aspect, the therapeutically effective amounts for oral administration is from about 1 mg/day to about 100 mg/day; and for parenteral administration from about 5 to about 50 mg daily. In a more preferred aspect, the therapeutically effective amounts for oral administration are from about 5 mg/day to about 50 mg/day.

Compound of the present invention can be administered orally using any pharmaceutically acceptable dosage form known in the art for such administration. The vehicle may be any solution, suspension, powder, gel, etc., including isotonic solution, buffered and saline solutions, such as syrups or aqueous suspensions, etc. The compounds may be administered by any suitable route, including systemic delivery, intra-venous, intra-arterial, intra-cerebral or intrathecal injections. Repeated injections may be performed, if desired. The dosage can vary within wide limits and will have to be adjusted to the individual requirements in each particular case, depending upon several factors known to those of ordinary skill in the art. Agents determining the dosage of dosage the active compounds can be the pharmacodynamic characteristics of the particular agent and its mode and route of administration; the age, health and weight of the recipient; the nature and extent of the symptoms; the kind of concurrent treatment; the frequency of treatment; and the effect desired. A daily dosage of active ingredient can be expected to be about 0.001 to about 1000 milligrams per kilogram of body weight, with the preferred dose being about 0.1 to about 30 mg/kg. The daily oral dosage can vary from about 0.01 mg to 1000 mg, 0.1 mg to 100 mg, or 10 mg to 500 mg per day of a compound. The daily dose may be administered as single dose or in divided doses and, in addition, the upper limit can also be exceeded when this is found to be indicated.

The compounds of the present invention can be administered in such oral dosage forms as tablets, capsules (each of which can include sustained release or timed release formulations), pills, powders, granules, elixirs, tinctures, suspensions, syrups, and emulsions. Likewise, they may also be administered in intravenous (bolus or infusion), intraperitoneal, subcutaneous, or intramuscular form, all using dosage forms well known to those of ordinary skill in the pharmaceutical arts. An effective but non-toxic amount of the compound desired can be employed to prevent or treat neurological disorders related to .beta.-amyloid production or accumulation, such as Alzheimer's disease and Down's Syndrome.

The compounds can be administered alone, but is generally administered with a pharmaceutical carrier, with respect to standard pharmaceutical practice (such as described in Remington's Pharmaceutical Sciences, Mack Publishing).

Compounds can be administered by any means that produces contact of the active agent with the agent's site of action in the body of a host, such as a human or a mammal. They can be administered by any conventional means available for use in conjunction with pharmaceuticals, either as individual therapeutic agents or in a combination of therapeutic agents, either administered alone, or administered with a pharmaceutical carrier selected on the basis of the chosen route of administration and standard pharmaceutical practice.

The compound for the present invention can be administered in intranasal form via topical use of suitable intranasal vehicles, or via transdermal routes, using those forms of transdermal skin patches wall known to those of ordinary skill in that art.

Oral administration in the form of a tablet or capsule containing the active compound can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents, and coloring agents can also be incorporated into the mixture. Sweetening agents such as those set forth above, and flavoring agents may be added to provide a palatable oral preparation. These compositions may be preserved by the addition of an anti-oxidant such as butylated hydroxyanisol or alpha-tocopherol. Suitable binders include starch, gelatin, natural sugars such as glucose or .beta.-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like.

Compounds can also be administered in the form of liposomal particulate delivery systems, such as small unilamellar vesicles, large unilamallar vesicles, and multilamellar vesicles. Liposomes can be formed from a variety of phospholipids, such as cholesterol, stearylamine, or phosphatidylcholines. Alternatively, compounds of the present invention may also be coupled with soluble polymers as targetable drug carriers, such as polymers made of polyvinylpyrrolidone, pyran copolymer, polyhydroxypropylmethacrylamide - phenol, polyhydroxyethylaspartamide - phenol, or polyethyleneoxide - polylysine substituted with palmitoyl residues. Polymers may also belong to the class of biodegradable polymers useful in achieving controlled release of a drug, for example, polylactic acid, polyglycolic acid, copolymers of polylactic and polyglycolic acid, polyepsilon caprolactone, polycyanoacylates, etc... or block copolymers of hydrogels.

Compounds of the present invention may be formulated into gelatin capsules with the addition of lactose, starch, cellulose derivatives, magnesium stearate, stearic acid, and the like as powdered carriers. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract. Liquid dosage forms for oral administration can contain coloring and flavoring to increase patient acceptance. In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propyl-paraben, and chlorobutanol.

The compounds of the invention can be used in the same manner, by the same routes of administration, using the same pharmaceutical dosage forms, and at the same dosing schedule as described above, for preventing disease or treating patients with MCI (mild cognitive impairment) and preventing or delaying the onset of Alzheimer's disease in those who would progress from MCI to AD, for treating or preventing Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, dementia associated with progressive supranuclear palsy, dementia associated with cortical basal degeneration, and diffuse Lewy body type of Alzheimer's disease. The compounds of the invention can be used with each other or with other agents used to treat or prevent the conditions listed above. Such agents include pharmaceutical agents such as donepezil hydrochloride (ARICEPT Tablets), tacrine hydrochloride (COGNEX Capsules), acetylcholine esterase inhibitors such as tacrine (tetrahydroaminoacridine, marketed as COGNEX(R)), donepezil hydrochloride, (marketed as Aricept(R) and rivastigmine (marketed as Exelon(R)); beta - or gamma-secretase inhibitors; anti-inflammatory agents such as cyclooxygenase II inhibitors; anti-oxidants such as Vitamin E; immunological approaches, such as, for example, immunization with Aβ peptide or administration of anti-Aβ peptide antibodies; statins; and direct or indirect neurotropic agents such as Cerebrolysin(R), and other neurotrophic agents. It should be apparent to one skilled in the art that the exact dosage and frequency of administration will depend on the particular compounds employed in the methods of the invention administered, the particular condition being treated, the severity of the condition being treated, the age, weight, general physical condition of the particular patient, and other medication the individual may be taking as is well known to administering physicians who are skilled in this art.

According to another aspect, the present invention relates to compounds for the treatment of patients who have, or in preventing patients from developing, a disease or condition of Alzheimer's disease, for helping prevent or delay the onset of Alzheimer's disease, for helping to slow the progression of Alzheimer's disease, for treating patients with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, dementia associated with progressive supranuclear palsy, dementia associated with cortical basal degeneration, or diffuse Lewy body type of Alzheimer's disease and who is in need of such treatment which comprises administration of a therapeutically effective amount of a compound of formula (I) as defined above.

In one aspect, this method of treatment can be used where the disease is Alzheimer's disease. In another aspect, this method of treatment can help prevent or delay the onset of Alzheimer's disease. In another aspect, this method of treatment can help slow the progression of Alzheimer's disease. In another aspect, this method of treatment can be used where the disease is mild cognitive impairment. In another aspect, this method of treatment can be used where the disease is Down's syndrome. In another aspect, this method of treatment can be used where the disease is Hereditary Cerebral Hemorrhage with Amyloidosis of the Dutch-Type. In another aspect, this method of treatment can be used where the disease is cerebral amyloid angiopathy. In another aspect, this method of treatment can be used where the disease is degenerative dementias. In another aspect, this method of treatment can be used where the disease is diffuse Lewy body type of Alzheimer's disease. In another aspect, this method of treatment can treat an existing disease, such as those listed above. In another aspect, this method of treatment can prevent a disease, such as those listed above, from developing or progressing.

The present invention also includes the use of at least one compound of formula (I), or a pharmaceutically acceptable salt thereof for the manufacture of a medicament for use in treating any Amyloid-β-Peptide Related Disorder. More specifically, the present invention deals with the use of at least one compound of formula (I), or a pharmaceutically acceptable salt thereof for the manufacture of a medicament for use in treating a patient who has, or in preventing a patient from developing, a disease or condition selected from the group consisting of Alzheimer's disease, for helping prevent or delay the onset of Alzheimer's disease, for treating patients with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, dementia associated with progressive supranuclear palsy, dementia associated with cortical basal degeneration, or diffuse Lewy body type of Alzheimer's disease.

In one aspect, this use of a compound of formula (I) can be employed where the disease is Alzheimer's disease. In another aspect, this use of a compound of formula (I) can help prevent or delay the onset of Alzheimer's disease. In another aspect, this use of a compound of formula (I) can help slow the progression of Alzheimer's disease. In another aspect, this use of a compound of formula (I) can be employed where the disease is mild cognitive impairment. In another aspect, this use of a compound of formula (I) can be employed where the disease is Down's syndrome. In another aspect, this use of a compound of formula (I) can be employed where the disease is Hereditary Cerebral Hemorrhage with Amyloidosis of the Dutch-Type. In another aspect, this use of a compound of formula (I) can be employed where the disease is cerebral amyloid angiopathy. In another aspect, this use of a compound of formula (I) can be employed where the disease is degenerative dementias.

The present invention also includes *in vitro, ex vivo* or *in vivo* methods for inhibiting cleavage of amyloid precursor protein (APP), in a reaction mixture, at a site relevant to the production of amyloid beta peptide (Aβ) in a cell; for inhibiting the production of beta-amyloid plaque in an animal; and for treating or preventing a disease characterized by beta-amyloid deposits in the brain. These methods each include administration of a therapeutically effective amount of a compound of formula (I), or a pharmaceutically acceptable salt thereof.

The present invention also includes an *in vitro, ex vivo* or *in vivo* method for inhibiting beta- or gamma-secretase mediated cleavage of APP, including exposing said beta-secretase or gamma-secretase to an effective inhibitory amount of a compound of formula (I), or a pharmaceutically acceptable salt thereof, including modifying the likelihood of APP for being cut by said beta-secretase or gamma-secretase. In one aspect, this method includes exposing said beta-secretase or gamma-secretase to said compound in vitro, in a cell or in an animal. In another aspect, this method includes exposing said beta-secretase or gamma-secretase to said compound in a human.
The present invention also includes a method for inhibiting cleavage of APP at a site relevant to the production of amyloid beta peptide (Aβ) in the APP-695 amino acid isotype; or at a corresponding site of an isotype or mutant thereof, including the APP-751 isotype, the APP-770 isotype; the APP-695 Swedish Mutation; the APP-751 Swedish Mutation; or the APP-770 Swedish Mutation, by using an effective inhibitory amount of a compound of formula (I), or a pharmaceutically acceptable salt thereof. The present invention also includes a method for inhibiting cleavage of APP bearing the Dutch, Artic, Flemish, Iowa, or Italian mutations.

The present invention provides compounds, compositions, kits, and methods for inhibiting cleavage of amyloid precursor protein (APP). The present invention also includes an agent kit including a compound of formula (I), or a pharmaceutically acceptable salt thereof; and one or more therapeutic agents selected from the group consisting of an antioxidant, an anti-inflammatory, a beta secretase inhibitor, a gamma secretase inhibitor, a neurotrophic agent, an acetyl cholinesterase inhibitor, a statin, an Aβ peptide, and an anti-Aβ antibody. More particularly, the compounds, compositions, and methods of the invention are effective to inhibit the production of Aβ peptide and to treat or prevent any human or veterinary disease or condition associated with a pathological form of Aβ peptide. The compounds, compositions, and methods of the invention are particularly useful for treating humans presenting Alzheimer's Disease (AD), for helping prevent or delay the onset of AD, for treating patients with mild cognitive impairment (MCI), and preventing or delaying the onset of AD in those patients who would otherwise be expected to progress from MCI to AD, for treating Down's syndrome, for treating Hereditary Cerebral Hemorrhage with Amyloidosis of the Dutch Type, for treating cerebral beta-amyloid angiopathy and preventing its potential consequences such as single and recurrent lobar hemorrhages, for treating other degenerative dementias, including dementias of mixed vascular and degenerative origin, for treating dementia associated with Parkinson's disease, dementia associated with progressive supranuclear palsy, dementia associated with cortical basal degeneration, and diffuse Lewy body type AD.

The present invention provides methods and kits for inhibiting APP cleavage and Aβ peptide production and/or for inhibiting the likelihood of APP of being cut by beta- and/or gamma-secretase. Inhibition of beta-secretase or gamma- secretase - mediated cleavage of APP halts or reduces the production of Aβ from APP and reduces or eliminates the formation of beta-amyloid deposits in the brain. The compounds of the invention and pharmaceutically acceptable salts thereof, are useful for treating subject (humans or animals) suffering from a condition characterized by a pathological form of beta-amyloid peptide, such as beta-amyloid plaques, and for helping to prevent or delay the onset of such a condition. For example, the compounds are useful for treating Alzheimer's disease, for helping prevent or delay the onset of Alzheimer's disease, for treating patients with MCI (mild cognitive impairment) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobal hemorrhages, for treating other degenerative dementias, including dementias of mixed vascular and degenerative origin, dementia associated with Parkinson's disease, dementia associated with progressive supranuclear palsy, dementia associated with cortical basal degeneration, and diffuse Lewy body type Alzheimer's disease. The compounds and compositions of the invention are particularly useful for treating, preventing, or slowing the progression of Alzheimer's disease. When treating or preventing these diseases, the compounds of the invention can either be used individually or in combination, as is best for the patient.

With regard to these diseases, the term "treating" means that compounds of the invention can be used in humans with existing disease, including at early or late stages of progression of the disease. The compounds of the invention will not necessarily cure the patient who has the disease but will delay or slow the progression or prevent further progression of the disease thereby giving the individual a more useful life span, in particular by reducing Aβ peptide production or accumulation, ameliorating the patients' condition, etc. Treatment can be used alone or in combination with other active agents.
The term "preventing" means that if the compounds of the invention are administered to those who do not have the diseases but who would normally develop the disease or be at increased risk for the disease, they will not develop the disease. In addition, "preventing" also includes delaying the development of the disease in an individual who will ultimately develop the disease or would be at risk for the disease due to age, familial history, genetic or chromosomal abnormalities, and/or due to the presence of one or more biological markers for the disease, such as a known genetic mutation of APP or APP cleavage products in brain tissues or fluids. By delaying the onset of the disease, compounds of the invention have prevented the individual from getting the disease during the period in which the individual would normally have gotten the disease or reduce the rate of development of the disease or some of its effects but for the administration of compounds of the invention up to the time the individual ultimately gets the disease. Preventing also includes administration of the compounds of the invention to those individuals thought to be predisposed to the disease. In a preferred aspect, the compounds of the invention are useful for slowing the progression of disease symptoms. In another preferred aspect, the compounds of the invention are useful for preventing the further progression of disease symptoms. In treating or preventing the above diseases, the compounds of the invention are administered in a therapeutically effective amount.

The therapeutically effective amount will vary depending on the particular compound used and the route of administration, as is known to those skilled in the art. In treating a patient displaying any of the diagnosed above conditions a physician may administer a compound of the invention immediately and continue administration indefinitely, as needed. In treating patients who are not diagnosed as having Alzheimer's disease, but who are believed to be at substantial risk for Alzheimer's disease, the physician should preferably start treatment when the patient first experiences early pre-Alzheimer's symptoms such as, memory or cognitive problems associated with aging. In addition, there are some patients who may be determined to be at risk for developing Alzheimer's through the detection of a genetic marker such as APOE4 or other biological indicators that are predictive for Alzheimer's disease. In these situations, even though the patient does not have symptoms of the disease, administration of the compounds of the invention may be started before symptoms appear, and treatment may be continued indefinitely to prevent or delay the onset of the disease.

### Inhibition of APP Cleavage

While not wishing to be bound by a particular theory, inhibition of APP cleavage may involve inhibition of beta-secretase activity or of gamma-secretase activity, or decrease in the likelihood of APP of being cut by beta-secretase and/or of gamma-secretase which results in the inhibition of beta amyloid peptide (Aβ) production. Inhibitory activity is demonstrated in one of a variety of inhibition assays, whereby cleavage of an APP substrate in the presence of beta-secretase and gamma-secretase activities is analyzed in the presence of the inhibitory compound, under conditions normally sufficient to result in cleavage at the beta-secretase and gamma-secretase cleavage sites. Reduction of APP cleavage compared with an untreated or inactive control is correlated with inhibitory activity. Assay systems that can be used to demonstrate efficacy of the compound inhibitors of the invention are known. Representative beta-secretase assay systems are described, for example, in U.S. Pat. Nos. 5,942,400 and 5,744,346. Representative gamma-secretase assay systems are described, for example in Pinnix et al. (2001) J. Biol. Chem. 276, 481-487; Li et al. (2000) Proc. Natl. Acad. Sci. U. S. A. 97, 6138-6143. The production of Aβ can be analyzed *in vitro* or *in vivo,* using natural, mutated, and/or synthetic APP substrates, natural, mutated, and/or synthetic enzyme, and the test compound. The analysis may involve primary or secondary cells expressing native, mutant, and/or synthetic APP and enzyme, animal models expressing native APP and enzyme, or may utilize transgenic animal models expressing the substrate and enzyme. Detection of enzymatic activity can be by analysis of one or more of the cleavage products, for example, by immunoassay, fluorometric or chromogenic assay, HPLC, or other means of detection. Inhibitory compounds are determined as those having the ability to decrease the amount of beta-secretase cleavage product produced in comparison to a control performed in the absence of inhibitory compounds.

### Antibodies

Products characteristic of APP cleavage can be measured by immunoassay using various antibodies, as described, for example, in Pirttila et al., 1999, Neuro. Lett. 249:21-4 and in U.S. Pat. No. 5,612,486. Useful antibodies to detect Aβ include, for example, the monoclonal antibody 6E10 (Biosource, Camarillo, Ca.) that specifically recognizes an epitope on amino acids 1-16 of the Aβ peptide; and antibodies that are specific for human Aβ 1-40 and 1-42. Antibody raised against a synthetic peptide of residues 737 to 751 of APP is also useful in immunoassay of APP and its cleavage products, such as the anti-APP
Cter (Serotec, Oxford, UK).

### Assay Systems

### Cell Free Assays

Exemplary assays that can be used to demonstrate the inhibitory activity of the compounds of the invention are described, for example, in WO00/17369, WO 00/03819, and U.S. Pat. Nos. 5,942,400 and 5,744,346. Such assays can be performed in cell-free incubations or in cellular incubations using cells expressing a beta-secretase and an APP substrate having a beta-secretase cleavage site, a gamma-secretase and an APP substrate having a gamma-secretase cleavage site or a combination of both., provided that APP is cleaved at both beta-secretase and gamma-secretase cleavage sites to produce Aβ. An APP substrate containing the beta- or gamma cleavage site of APP, for example, a complete APP or variant, an APP fragment, or a recombinant or synthetic APP substrate containing the beta- or gamma cleavage site is incubated in the presence of a beta-secretase, a gamma-secretase or both, a fragment thereof, or a synthetic, purified or recombinant polypeptide variant having beta-secretase or gamma-secretase activity and effective to cleave beta- or gamma cleavage site of APP, under incubation conditions suitable for the cleavage activity, provided that APP is cleaved at both beta-secretase and gamma-secretase cleavage sites to produce Aβ. Suitable substrates optionally include derivatives that may be fusion proteins or peptides that contain the substrate peptide and a modification useful to facilitate the purification or detection of the peptide or its cleavage products. Useful modifications include the insertion of a known antigenic epitope for antibody binding; the linking of a label or detectable moiety, the linking of a binding substrate, and the like. Suitable incubation conditions for a cell-free in vitro assay include, for example: approximately 200 nanomolar to 10 micromolar substrate, approximately 10 to 200 picomolar enzyme, and approximately 0.1 nanomolar to 10 micromolar inhibitor compound, in aqueous solution, at an approximate pH of 4-7, at approximately 37 degrees C, for a time period of approximately 10 minutes to 3 hours. These incubation conditions are exemplary only, and can be varied as required for the particular assay components and/or desired measurement system. Optimization of the incubation conditions for the particular assay components should account for the specific secretase enzyme used and its pH optimum, any additional enzymes and/or markers that might be used in the assay, and the like. Such optimization is routine and will not require undue experimentation. Analysis of the cleavage activity can be, for example, by immunoassay of cleavage products.

### Cellular Assay

Numerous cell-based assays can be used to analyze secretase activity and/or processing of APP to release Aβ. Contact of an APP substrate with a beta-secretase or gamma-secretase enzyme within the cell and in the presence or absence of a compound inhibitor of the invention can be used to demonstrate secretase inhibitory activity of the compound. Preferably, assay in the presence of a useful inhibitory compound provides at least about 30%, most preferably at least about 50% inhibition of the enzymatic activity, as compared with a non-inhibited control. In one embodiment, cells that naturally express beta-secretase or gamma-secretase are used. Alternatively, cells are modified to express a recombinant beta-secretase or gamma-secretase or the combination of the two, or synthetic variant enzymes. The APP substrate is preferably expressed in the cells. Cells that naturally express APP, variant or mutant forms of APP, or cells transformed to express an isoform of APP, mutant or variant APP, recombinant or synthetic APP, APP fragment, or synthetic APP peptide or fusion protein containing the beta-and gamma- secretase APP cleavage sites can be used, provided that the expressed APP is permitted to contact the enzyme and enzymatic cleavage activity can be analyzed. Human cell lines that normally process Aβ from APP provide a useful means to assay inhibitory activities of the compounds of the invention. Production and release of Aβ and/or other cleavage products into the culture medium can be measured, for example by immunoassay, such as Western blot or enzyme-linked immunoassay (EIA) such as by ELISA. Inhibition of secretase activity against the substrate APP would be expected to decrease release of specific secretase induced APP cleavage products such as Aβ. The use of cell types known to have enhanced beta-secretase or gamma-secretase activity, enhanced processing of APP to Aβ, and/or enhanced production of Aβ are preferred. For example, transfection of cells with the Swedish Mutant form of APP (APP-SW) provides cells having enhanced secretase activity and producing amounts of Aβ that can be readily measured. In such assays, for example, the cells are incubated in a culture medium under conditions suitable for beta-secretase and gamma-secretase enzymatic activity at its respective cleavage site on the APP substrate. On exposure of the cells to the compound inhibitor, the amount of Aβ released into the medium and/or the amount of CTF99 fragment of APP in the cell lysates is enhanced as compared to the control. The cleavage products of APP can be analyzed, for example, by immune reactions with specific antibodies, as discussed above. Preferred cells for analysis of APP processing by beta-secretase and gamma-secretase activity include primary human neuronal cells, primary transgenic animal neuronal cells where the transgene is APP, and other cells such as those of a stable 293 cell line expressing APP, for example, APP-SW.

### In Vivo Assays

### Animal Models

Various animal models can be used to analyze beta-secretase activity and/or processing of APP to release Aβ, as described above. For example, transgenic animals expressing APP substrate and beta-secretase or gamma-secretase enzymes can be used to demonstrate inhibitory activity of the compounds of the invention. Certain transgenic animal models have been described, for example, in U.S. Pat. Nos. 5,877,399; 5,612,486; 5,387,742; 5,720,936; 5,850,003; 5,877,015, and 5,811,633, and in Ganes et al., 1995, Nature 373:523. Preferred animals are those which exhibit characteristics associated with the pathophysiology of AD. However, the guinea pig model is also a useful model because it produce high levels of Aβ 40 and 42 peptides which are similar (i.e. same amino acid structure) to that generated from human APP and readily detectable using antibodies raised against human Aβ. Advantagenously, in contrast to transgenic mice described herein, the guinea pig model represent a physiological model of APP processing and Aβ production, although it does not present amyloid plaks, nor cognitive impairment due to high Aβ levels.
Administration of the compound inhibitors of the invention to the transgenic mice and guinea pigs described herein provides an alternative method for demonstrating the inhibitory activity of the compounds. Administration of the compounds in a pharmaceutically effective carrier and via an administrative route that reaches the target tissue in an appropriate therapeutic amount is also preferred. Inhibition of APP processing and of Aβ release can be analyzed in these animals by measure of cleavage fragments in the animal's body fluids such as cerebral fluid or tissues. Analysis of brain tissues for reduced Aβ deposits or plaques is preferred. The compounds of the invention are effective if they are demonstrated to reduce Aβ levels, Aβ deposition in brain tissues, and to reduce the number and/or size of beta amyloid plaques. In human subject, the compounds are effective to inhibit or slow the progression of disease characterized by enhanced amounts of Aβ, to slow the progression of AD in the, and/or to prevent onset or development of AD in a patient at risk for the disease. Unless defined otherwise, all scientific and technical terms used herein have the same meaning as commonly understood by one of skill in the art to which this invention belongs. All patents and publications referred to herein are hereby incorporated by reference for all purposes

Further aspects and advantages of this invention will be disclosed in the following examples, which should be regarded as illustrative and not limiting the scope of this application.

### EXAMPLES

### Examples 1 to 16 disclose the synthesis and physico-chemical properties of compounds according to this invention.

### General

¹H NMR spectra were recorded at ambient temperature with an Advance 300 (Bruker) spectrometer. The compounds were analyzed by reverse phase high performance liquid chromatography (HPLC) using a Waters Autopurification System equipped with a Waters 2525 Pump, a Waters 2696 photodiode array detector, and a XTerra column (Part. No. 186000482, 5 µm, C18, 4.5 × 50 mm). The HPLC method used was a gradient of solvent B:solvent A = 5:95 to 100 % solvent B in 7 min. Solvent A was H₂O with 0.05 % TFA and solvent B was CH₃CN with 0.05 % TFA (Method A). Melting points were measured with a Büchi B-545 melting point apparatus and were uncorrected. To isolate reaction products the solvent were removed by evaporation using a vacuum rotatory evaporator, the water bath temperature not exceeding 40 °C except for *N,N*-dimethylformamide removal.

### Preparation of 4-(5-bromopentylthio)-7-(trifluoromethyl)quinoline.

7-Trifluoromethyl-4-quinoline-thiol (1.0 g, 4.4 mmol), 1,5-dibromopentane (2.9 mL, 20.0 mmol) and CHCl₃ (16 mL) were charged in a 50 ml round-bottomed flask equipped with a magnetic stirrer. *n*-Tetrabutylammonium bromide (140 mg, 0.4 mmol) and water (4 mL) were added and the orange reaction mixture was stirred for 36 h at RT (yellow reaction mixture). The organic phase was evaporated at 30 °C to dryness to give an oily crude product (5.5 g). The crude product was dissolved in EtOAc (25 mL) and a solution of HCl 0.4 M in Et₂O (11 mL) was added. Evaporation at 30 °C to dryness led to a solid crude product contaminated with 1,5-dibromopentane (6.1 g). After washing the crude product with cyclohexane (3 x 50 mL) to get an odourless solid, the compound was filtered to give 1.6 g of pale yellow product and was stirred in 220 mL of EtOAc for 1 h. The solution was filtered off and the filtrate was washed with 2 N aqueous cold HCl (50 mL), water (2 x 20 mL), saturated aqueous Na₂CO₃ (2 x 20 mL), water (2 x 20 mL), brine (20 mL), dried over MgSO₄, filtered and evaporated to dryness. 4-(5-Bromopentylthio)-7-(trifluoromethyl)quinoline (0.85 g, 52 % yield) was obtained as a pale yellow solid. 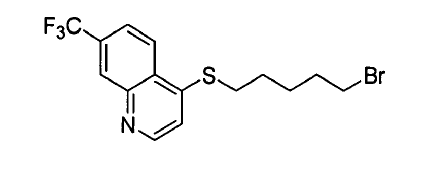 MW: 378.25; Yield: 52 %; Pale Yellow Solid; Mp (°C): 60.8
R_{f}: 0.75 (CH₂Cl₂ :EtOAc = 9:1).
¹H-NMR (CDCl₃, δ): 1.67-1.75 (m, 2H, CH₂), 1.81-1.99 (m, 4H, CH₂), 3.14 (t, 2H, *J* = 7.2 Hz, S-CH₂), 3.44 (t, 2H, *J =* 6.6 Hz, N-CH₂), 7.25 (d, 1H, *J =* 4.8 Hz, Ar-H), 7.71 (dd, 1H, *J* = 1.8 Hz, *J* = 8.8 Hz, Ar-H), 8.23 (d, 1H, *J* = 8.8 Hz, Ar-H), 8.36 (s, 1H, Ar-H), 8.79 (d, 1H, *J=* 4.8 Hz, Ar-H).
MS-ESI m/z (rel. int.): 378.0-379.8 ([MH]⁺, 93-100).
HPLC: Method A, detection UV 254 nm, RT = 6.0 min, peak area 99.9 %.

### Example 1: 4-(5-(4-((piperazin-1-yl)methyl)phenoxy)pentylthio)-7-(trifluoromethyl) quinoline trihydrochloride 1.

### tert-Butyl 4-(4-hydroxybenzyl)piperazine-1-carboxlate.

To a solution of 4-hydroxybenzaldehyde (2.29 g, 18.79 mmol) in MeOH (50 mL) was added *tert*-butyl piperazine-1-carboxylate (3.50 g, 18.79 mmol) in a 100 mL round-bottomed flask equipped with a magnetic stirrer. The reaction mixture was stirred for 2 h at RT. NaCNBH₃ (1.41 g, 22.55 mmol) was added and the reaction mixture was stirred at RT for 20 h. MeOH was evaporated and EtOAc (100 mL) was added. The precipitate was filtered off and ethyl acetate was evaporated at 40 °C to dryness. The crude compound was purified by column chromatography (SiO₂, eluent EtOAc:cyclohexane = 40:60) to give after evaporation *tert*-butyl 4-(4-hydroxybenzyl)piperazine-1-carboxylate (950 mg, 17 % yield) as a yellow oil. 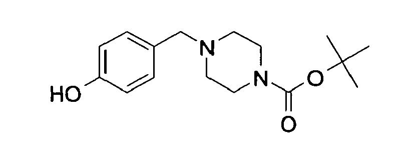 **MW:** 292.37; **Yield:** 17 %; Yellow Oil.
***R_{f}*:** 0.2 (EtOAc:cyclohexane = 40:60)
**¹H-NMR** (CDCl₃, δ): 1.45 (s, 9H, CH₃), 2.35-2.55 (m, 4H, N-CH₂), 3.30-3.60 (m broad, 6H, N-CH₂), 6.70 (d, 2H, *J=* 8.4 Hz, Ar-H), 7.10 (d, 2H, *J=* 8.4 Hz, Ar-H).
**MS-ESI** *m*/*z* (rel. int.): 293.1 ([MH]⁺, 15), 131.0 (100).
**HPLC:** Method A, detection UV 254 nm, RT = 4.0 min, peak area 82.1 %

### 4-(5-(4-((Piperazin-1-yl)methyl)phenoxy)pentylthio)-7-(trifluoromethyl)quinoline trihydrochloride 1.

*tert*-Butyl 4-(4-hydroxybenzyl)piperazine-1-carboxylate (0.95 g, 3.25 mmol), Cs₂CO₃ (1.1 g, 3.41 mmol) and anhydrous DMF (7 mL) were charged in a 100 ml round-bottomed flask equipped with a magnetic stirrer under inert atmosphere and the reaction mixture was stirred for 0.15 h at 50 °C. 4-(5-Bromopentylthio)-7-(trifluoromethyl)quinoline (1.29 g, 3.41 mmol) was added and the reaction mixture was stirred for 3 h at 90 °C. After evaporation of the DMF, the reaction mixture was poured in 50 mL of H₂O, extracted with EtOAc (2 x 200 mL). The organic layer was washed with brine (3 x 50 mL), dried over MgSO₄, filtered and evaporated to dryness. The crude product was purified by column chromatography (SiO₂, EtOAc:cyclohexane = 75:25) to give after evaporation *tert*-butyl 4-((4-(5-(7-(trifluoromethyl)quinolin-4-ylthio)pentyloxy)phenyl)methyl)piperazine-1-carboxylate as a white solid (750 mg, 40 % yield). This compound was dissolved in MeOH (50 mL) and a solution of HCl 0.5M in MeOH (25 mL, 12.71 mmol) was added and the reaction mixture was stirred for 3 h at 50 °C. After evaporation of MeOH the hydrochloride compound was dried under vacuum and triturated with EtOAc (50 mL) to give after filtration and drying 4-(5-(4-((piperazin-1-yl)methyl)phenoxy)pentylthio)-7-(trifluoromethyl)quinoline trihydrochloride **1** (683 mg, 36 % yield) as a pale yellow solid.

The structure of compound example 1 is presented below: 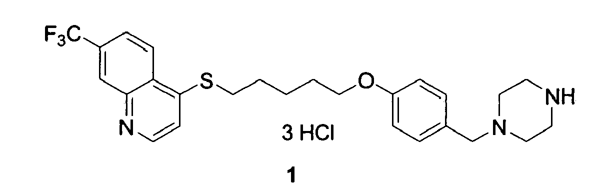 **MW:** 598.98; **Yield:** 40 %; Pale Yellow Solid; **Mp** (°C): 175.1
***R_{f}:*** 0.40 (CH₂Cl₂:MeOH = 95:5), free base.
**¹H-NMR** (CD₃OD, δ): 1.70-2.10 (m, 6H, CH₂), 3.35-3.90 (m, 10H, CH₂), 4.00-4.15 (t, 2H, *J =* 3.9 Hz, S-CH₂), 4.44 (s, 2H, O-CH₂), 7.02 (d, 2H, *J =* 8.5 Hz, Ar-H), 7.53 (d, 2H, *J =* 8.5 Hz, Ar-H), 8.04 (d, 1H, *J* = 6.3 Hz, Ar-H), 8.16 (d, 1H, *J* = 8.9 Hz, ArH), 8.47 (s, 1H, Ar-H), 8.68 (d, 1H, *J =* 8.8 Hz, Ar-H), 8.99 (d, 1H, *J =* 6.3 Hz, Ar-H).
**MS-ESI** *m*/*z* (rel. int.): 490.1 ([MH]⁺, 55), 298.1 (100).
**HPLC:** Method A, Detection UV 254 nm, RT = 4.38 min, peak area 99.9 %.

### General procedure for examples 2 to 4: Method B:

4-(5-(4-((piperazin-1-yl)methyl)phenoxy)pentylthio)-7-(trifluoromethyl)quinoline trihydrochloride 1 (0.13 g, 0.217 mmol) was charged in a 10 ml round-bottomed flask equipped with a magnetic stirrer. Anhydrous CHCl₃ (2 mL) and triethylamine (100 µL, 0.9 mmol) were successively added. After 30 min at 0 °C, carbamic or acyl chloride (0.26 mmol) in CHCl₃ (2 mL) was added slowly dropwise. The reaction mixture was stirred for 15 h at RT. The reaction mixture was poured in 10 mL of brine and extracted with CH₂Cl₂ (50 mL). The organic layer was dried over MgSO₄, filtered and evaporated to dryness. The crude product was purified by column chromatography (SiO₂) the desired free base. The obtained free base was dissolved in MeOH (2 mL) in a 25 mL round-bottomed flask equipped with a magnetic stirrer. A solution of HCl 0.5 M in MeOH (2 mL) was added dropwise at RT. The reaction mixture was stirred for 2 h at RT. The reaction mixture was evaporated *in vacuo.* MeOH (3x30 mL) was added then evaporated. The hydrochloride compound was dried under vacuum and triturated in EtOAc (200 mL) to give after filtration and drying the desired end product.

### Example 2 : 4-((4-(5-(7-(trifluoromethyl)quinolin-4-ylthio)pentyloxy)phenyl)methyl)-N,N-diethylpiperazine-1-carboxamide dihydrochloride 2.

The compound was prepared according to method B with diethylcarbamic chloride (35 mg, 0.26 mmol). After purification by column chromatography (eluent EtOAc:MeOH = 95:5) the desired free base (96.8 mg, 76 % yield) was obtained. Salt formation led to 4-((4-(5-(7-(trifluoromethyl)quinolin-4-ylthio)pentyloxy)phenyl)methyl)-*N,N*-diethylpiperazine-1-carboxamide dihydrochloride **2** (33 mg, 21 % yield) as a pale yellow solid.

The structure of compound example 2 is presented below: 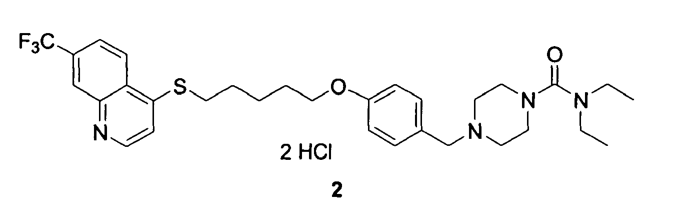 **MW:** 661.68; **Yield:** 21 %; Pale Yellow Solid; **Mp** (°C): 182.2
**¹H-NMR** (CD₃OD, δ): 1.14 (t, 6H, *J* = 8.6 Hz, CH₃), 1.70-1.83 (m, 2H, CH₂), 1.83-2.05 (m, 4H, CH₂), 3.01-3.49 (m, 12H, CH₂), 3.68 (m, 2H, N-CH₂), 4.06 (t, 2H, *J =* 4.5 Hz, S-CH₂), 4.30 (s, 2H, O-CH₂), 7.02 (d, *J =* 8.4 Hz, 2H, Ar-H), 7.43 (d, 2H, *J* = 8.4 Hz, Ar-H), 7.81 (d, 1H, *J =* 5.4 Hz, Ar-H), 8.01 (d, 1H, *J* = 8.8 Hz, Ar-H8.37 (s, 1H, Ar-H), 8.54 (d, 1H, *J* = 8.6 Hz, Ar-H), 8.88 (d, 1H, *J* = 5.6 Hz, Ar-H).
**MS-ESI** *m*/*z* (rel. int.): 589.1 ([MH]⁺, 80), 298.0 (100).
**HPLC:** Method A, Detection UV 254 nm, RT = 5.07 min, peak area 99.9 %.

### Example 3: 1-(4-((4-(5-(7-(trifluoromethyl)quinolin-4-ylthio)pentyloxy)phenyl)methyl) piperazin-1-yl)-2,2-dimethylpropan-1-one dihydrochloride 3.

The compound was prepared according to method B with pivaloyl chloride (31 mg, 0.26 mmol). After purification by column chromatography (eluent EtOAc:cyclohexane = 9:1) the desired free base (100.6 mg, 80 % yield) was obtained. Salt formation led to 1-(4-((4-(5-(7-(trifluoromethyl)quinolin-4-ylthio)pentyloxy)phenyl)methyl)piperazin-1-yl)-2,2-dimethylpropan-1-one dihydrochloride 3 (33 mg, 45 % yield) as a pale yellow solid.

The structure of compound example 3 is presented below: 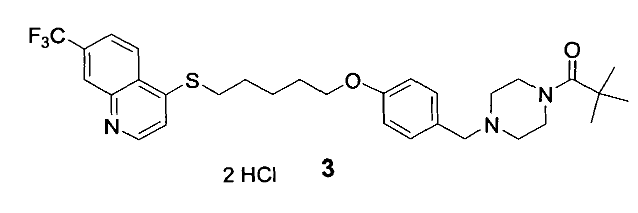 **MW:** 646.63; **Yield:** 45 %; Pale Yellow Solid; **Mp** (°C): 128.0
**¹H-NMR** (CD₃OD, δ): 1.28 (s, 9H, CH₃), 1.68-1.82 (m, 2H, CH₂), 1.82-2.02 (m, 4H, CH₂), 3.10-3.60 (m, 10H, CH₂), 4.05 (t, 2H, *J=* 4.8 Hz, S-CH₂), 4.29 (s, 2H, O-CH₂), 7.02 (d, *J =* 8.5 Hz, 2H, Ar-H), 7.42 (d, 2H, *J* = 8.3 Hz, Ar-H), 7.65 (d, 1H, *J =* 5.2 Hz, Ar-H), 7.90 (d, 1H, *J* = 8.7 Hz, Ar-H), 8.31 (s, 1H, Ar-H), 8.43 (d, 1H, *J* = 8.6 Hz, Ar-H), 8.79 (d, 1H, *J =* 5.1 Hz, Ar-H).
**MS-ESI** *m*/*z* (rel. int.): 574.1 ([MH]⁺, 60), 298.0 (100).
**HPLC:** Method A, Detection UV 254 nm, RT = 5.07 min, peak area 98.2 %.

### Example 4: 4-((4-(5-(7-trifluoromethyl)quinolin-4-ylthio)pentyloxy)phenyl)methyl)-N,N-diisopropylpiperazine-1-carboxamide dihydrochloride 4.

The compound was prepared according to method A with diisopropylcarbamic chloride (35 mg, 0.26 mmol). After purification by column chromatography (eluent EtOAc:cyclohexane = 9:1) the desired free base (107 mg, 80% yield) was obtained. Salt formation led to 4-((4-(5-(7-(trifluoromethyl)quinolin-4-ylthio)pentyloxy)phenyl)methyl)-*N,N*-diisopropylpiperazine-1-carboxamide dihydrochloride **4** (30 mg, 21 % yield) as a white solid.

The structure of compound example 4 is presented below: 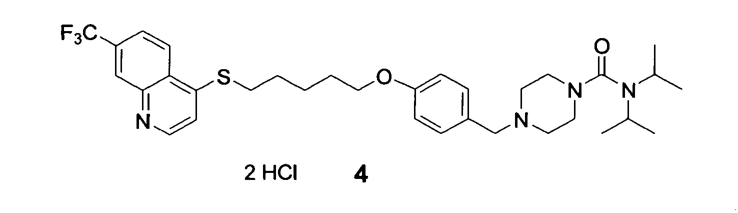 **MW:** 689.7; **Yield:** 21 %; White Solid; **Mp:** too hygroscopic.
**¹H-NMR** (CD₃OD, δ): 1.28 (m, 12H, CH₃), 1.70-1.85 (m, 2H, CH₂), 1.85-2.10 (m, 4H, CH₂), 2.95-3.22 (m, 4H, CH₂), 3.32-3.62 (m, 6H, CH₂), 3.62-3.82 (m, 2H, CH₂), 4.06 (t, 2H, *J =* 3.9 Hz, S-CH₂), 4.30 (s, 2H, O-CH₂), 7.01 (d, 2H, *J =* 5.1 Hz, Ar-H), 7.47 (d, 2H, 5.3 Hz, Ar-H), 8.01 (s, 1H, Ar-H), 8.14 (d, 1H, *J* = 8.8 Hz, Ar-H), 8.47 (s, 1H, Ar-H), 8.67 (d, 1H, *J =* 6.1 Hz, Ar-H), 8.99 (s, 1H, Ar-H).
**MS-ESI** *m*/*z* (rel. int.): 617.2 ([MH]⁺, 100).
**HPLC:** Method A, Detection UV 254 nm, RT = 5.35 min, peak area 99.9 %.

### Example 5: 4-(5-(4-((piperidin-1-yl)methyl)phenoxy)pentylthio-7-(trifluoromethyl)quinoline dihydrochloride 5.

### 4-((Piperidin-1-yl)methyl)phenol.

To a stirred solution of 4-hydroxybenzaldehyde (6.11 g, 0.05 mmol) in piperidine (7.33 mL, 0.10 mmol) was added *via* syringe formic acid (4.9 mL, 0.13 mmol). The mixture was stirred for 2 h at 115 °C and after cooling poured in water (300 mL). The solution was acidified by a solution of aqueous HCl (1 M, 40 mL) and extracted with diethyl ether (4 x 100 mL). The aqueous solution was basified with a solution of NH₄OH (20% water, 14 mL). The white precipitate was filtered off and washed well with water and hexane. After drying, 4-((piperidin-1-yl)methyl)phenol (5.82 g, 61 % yield) was obtained as a white solid. 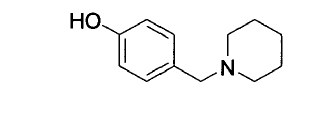 **MW:** 191.27; **Yield:** 61 %; White Solid; **Mp** (°C): 142.1 (dec).¹H-NMR (CDCl₃, δ): 1.44 (m, 2H, CH₂), 1.57-1.65 (m, 4 H, CH₂), 2.50 (s broad, 4H, N-CH₂), 3.40 (s, 2H, N-CH₂), 6.51 (d, 2H, *J* = 8.4 Hz, Ar-H), 7.03 (d, 2H, *J* = 8.4 Hz, Ar-H), OH not seen.
MS-ESI *m*/*z* (rel. int.): 192.2 ([MH]⁺, 20), 107.0 (100).

### 4-(5-(4-((Piperidin-1-yl)methyl)phenoxy)pentylthio)-7-(trifluoromethyl)quinoline dihydrochloride 5.

4-((Piperidin-1-yl)methyl)phenol (1.06 g, 5.29 mmol) was dissolved in DMF (10 mL) and cesium carbonate (1.81 g, 5.55 mmol) was added under a nitrogen atmosphere. The reaction mixture was stirred at 50 °C for 0.4 h. A solution of 4-(5-bromopentylthio)-7-(trifluoromethyl)quinoline (2.00 g, 5.55 mmol) in 5 mL of DMF was added *via* syringe. The organic mixture was stirred under nitrogen at 85 °C for 3 h. After cooling the mixture to room temperature DMF was evaporated. 50 mL of H₂O were added to the reaction mixture which was extracted with EtOAc (2 x 100 mL). The organic layer was washed with water (3 x 50 mL) and brine (2 x 50 mL), dried over MgSO₄, filtered and evaporated.

The crude product was purified by column chromatography on silica using as eluent CH₂Cl₂:MeOH = 95:5. After evaporation and drying a white solid 4-(5-(4-((piperidin-1-yl)methyl)phenoxy)pentylthio)-7-(trifluoromethyl)quinoline was obtained (2.65 g, 98 % yield).
The product was dissolved in ethanol (5 mL) and this solution was stirred at RT. Aqueous HCl (1 M, 120 mL) was added *via* syringe at RT for 1 h. The solution was evaporated to dryness and triturated with EtOAc. After filtration and drying 4-(5-(4-((piperidin-1-yl)methyl)phenoxy)pentylthio)-7-(trifluoromethyl)quinoline dihydrochloride **5** was obtained (1.16 g, 37 % yield) as a white solid.

The structure of compound example 5 is presented below: 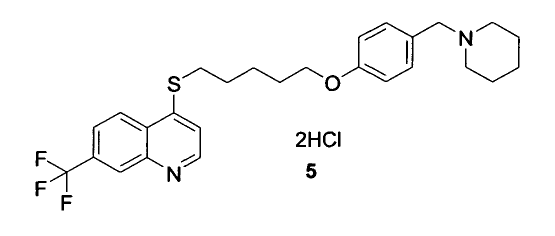 **MW:** 561.63; **Yield:** 38 %; White Solid; **Mp** (°C): 51.2
**R*_{f}*:** 0.30 (MeOH:CH₂Cl₂ = 10:90), free base.
**¹H-NMR** (CD₃OD, δ): 1.45-2.02 (m, 12H, CH₂), 2.89-2.97 (t, 2H, *J=* 11.5 Hz, S-CH₂), 3.30-3.44 (m, 4H, CH₂), 4.03-4.11 (t, 2H, *J=* 5.2 Hz, CH₂), 4.22 (s, 2H, CH₂), 6.99 (d, 2H, *J=* 8.5 Hz, Ar-H), 7.43 (d, 2H, *J=* 8.5 Hz, Ar-H), 7.63 (d, 1H, *J=* 5.1 Hz, Ar-H), 7.88 (d, 1H, *J* = 8.8 Hz, ArH), 8.30 (s, 1H, Ar-H), 8.40 (d, 1H, *J* = 8.8 Hz, Ar-H), 8.78 (d, 1H, *J* = 5.1 Hz, Ar-H).
**MS-ESI** m/z (% rel. Int.): 489.0 ([MH]⁺, 45), 404.0 (35), 298.0 (70), 106.9 (100).**HPLC:**
Method A, detection UV 254 nm, RT = 5.03 min, peak area 99.9 %.

### Example 6: 4-(5-(2-methyl-4-((piperidin-1-yl)methyl)phenoxy)pentylthio)-7-(trifluoromethyl)quinoline dihydrochloride 6.

### 2-Methyl-4-((piperidin-1-yl)methyl)phenol

To a solution of 4-hydroxy-3-methylbenzaldehyde (1.08 g, 7.90 mmol) in MeOH (20 mL) was added piperidine (0.8 mL, 7.9 mmol) in a 100 mL round-bottomed flask equipped with a magnetic stirrer. The reaction mixture was stirred for 2 h at RT. NaCNBH₃ (0.6 g, 9.5 mmol) was added and the reaction mixture was stirred at RT for 24 h. MeOH was evaporated and EtOAc (100 mL) was added. The precipitate was filtered off and EtOAc was evaporated at 40 °C to dryness. The crude compound was purified by column chromatography (SiO₂; eluent CH₂Cl₂:MeOH = 98:2 to 95:5) to give after evaporation 2-methyl-4-((piperidin-1-yl)methyl)phenol (800 mg, 48 % yield) as a beige solid. 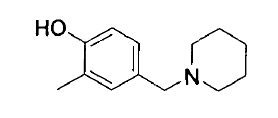 **MW:** 205.30; **Yield:** 48 %; Beige Solid; Mp (°C) = 117.2
*R_{f}.* 0.47 (CH₂Cl₂:MeOH = 9:1).
**¹H-NMR** (CDCl₃, δ): 1.35-1.45 (m, 2H, CH₂), 1.55-1.62 (m, 4H, CH₂), 2.16 (s, 3H, CH₃), 2.44 (s broad, 4H, N-CH₂), 3.38 (s, 2H, N-CH₂), 6.41 (d, 1H, *J* = 8.1 Hz, Ar-H), 6.87 (d, 1H, *J* = 8.1 Hz, Ar-H), 7.00 (s, 1H, Ar-H), 7.94 (s broad, 1H, OH).
**¹³C-NMR** (CDCl₃, δ): 16.1, 24.2, 25.3, 54.2, 63.4, 114.9, 124.4, 127.8, 128.4, 132.7, 154.0. **MS-ESI** *m*/*z* (rel. int.): 206.1 ([MH]⁺, 15), 121.0 (100).
**HPLC:** Method A, detection UV 254 nm, RT = 3.5 min, peak area 95.0 %

### 4-(5-(2-Methyl-4-((piperidin-1-yl)methyl)phenoxy)pentylthio)-7-(trifluoromethyl)quinoline dihydrochloride 6.

2-methyl-4-((piperidin-1-yl)methyl)phenol (136 mg, 0.66 mmol), Cs₂CO₃ (215 mg, 0.66 mmol), NaI (40 mg, 0.66 mmol) and anhydrous DMF (3 mL) were charged in a 25 ml round-bottomed flask equipped with a magnetic stirrer under inert atmosphere. 4-(5-Bromopentylthio)-7-(trifluoromethyl)quinoline (250 mg, 0.66 mmol) was added and the reaction mixture was stirred for 3 h at 90 °C. After evaporation of DMF, the reaction mixture was poured in 50 mL of H₂O, extracted with EtOAc (2 x 100 mL). The organic layer was washed with brine (2 x 20 mL), dried over MgSO₄, filtered and evaporated to dryness. The crude compound was purified by column chromatography (SiO₂; eluent EtOAc:MeOH = 94:6) to give after evaporation a pure solid (230 mg,). The compound was dissolved in MeOH (3 mL), then a solution of HCl 1M in MeOH (1 mL, 1 mmol) was added and after evaporation of MeOH, the hydrochloride compound was dried under vacuum to yield to 4-(5-(2-methyl-4-((piperidin-1-yl)methyl)phenoxy)pentylthio)-7-(trifluoromethyl)quinoline dihydrochloride **6** (235 mg, 62 % yield) as a pale yellow powder.

The structure of compound example 6 is presented below: 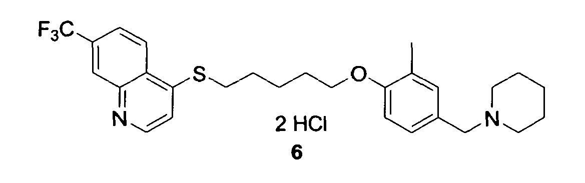 **MW:** 575.56; **Yield:** 62 %; Pale Yellow Powder; **Mp** (°C): 156.9
***R_{f}***: 0.20 (EtOAc:MeOH = 9:1), free base.
**¹H-NMR** (CD₃OD, δ): 1.47-2.08 (m, 12H, CH₂), 2.21 (s, 3H, CH₃), 2.92 (m, 2H, S-CH₂), 3.41 (m, 2H, N-CH₂), 3.53 (m, 2H, *J =* 7.2 Hz, N-CH₂), 4.08 (t, 2H, *J =* 5.9 Hz, O-CH₂), 4.18 (s, 2H, N-CH₂), 6.98 (d, 1H, *J* = 8.0 Hz, Ar-H), 7.26-7.33 (m, 2H, Ar-H) 8.00 (d, 1H, *J* = 6.4 Hz, Ar-H), 8.12 (d, 1H, *J* = 8.9 Hz, Ar-H), 8.46 (s, 1H, Ar-H), 8.65 (d, 1H, *J =* 8.8 Hz, Ar-H), 8.98 (d, 1H, *J=* 6.1 Hz, Ar-H).
**MS-ESI** *m*/*z* (rel. int.): 503.2 ([MH]⁺, 55), 121.0 (100).
**HPLC:** Method A, detection UV 254 nm, RT = 5.2 min, peak area 98.3 %

### Example 7: 4-(5-(2-methoxy-4-((piperidin-1-yl)methyl)phenoxy)pentylthio)-7-(trifluoromethyl)quinoline dihydrochloride 7.

### 2-Methoxy-4-((piperidin-1-yl)methyl)phenol.

To a stirred solution of 2-(4-hydroxy-3-methoxyphenyl)acetaldehyde (7.61 g, 0.05 mmol) in piperidine (7.34 mL, 0.10 mmol) was added *via* syringe formic acid (4.9 mL, 0.13 mmol). The mixture was stirred for 2 h at 115 °C and after cooling poured in water (300 mL). The solution was acidified by a solution of aqueous HCl (1 M, 40 mL) and extracted with diethyl ether (4 x 100 mL). The aqueous solution was basified with a solution of NH₄OH (20% water, 14 mL). The white precipitate was filtered off and washed well with water and hexane. After drying 2-methoxy-4-((piperidin-1-yl)methyl)phenol (8.38 g, 76 % yield) was obtained as a white solid. 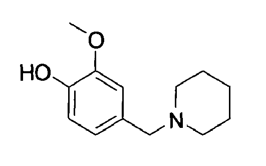 **MW:** 221.30; **Yield:** 76 %; White Solid; **Mp** (°C): 99.7 (dec).
**¹H-NMR** (CDCl₃, δ): 1.49 (s broad, 2H, CH₂), 1.60-1.67 (m, 4 H, CH₂), 2.52 (s, 4H, N-CH₂), 3.63 (s, 2H, N-CH₂), 3.83 (s, 3H, CH₃), 6.58 (dt, 2H, *J* = 1.4 Hz, *J* = 7.5 Hz, Ar-H), 6.72 (t, 2H, *J=* 7.5 Hz, Ar-H), 6.80 (dd, 2H, *J=* 1.4 Hz, *J=* 7.5 Hz, Ar-H), OH not seen.
**MS-ESI** *m*/*z* (rel. int.): 222.1 ([MH]⁺, 20), 137.1 (100).

### 4-(5-(2-Methoxy-4-((piperidin-1-yl)methyl)phenoxy)pentylthio-7-(trifluoromethyl)quinoline dihydrochloride 7.

2-Methoxy-4-((piperidin-1-yl)methyl)phenol (1.23 g, 5.29 mmol) was dissolved in DMF (10 mL) and cesium carbonate (1.82 g, 5.55 mmol) was added under a nitrogen atmosphere. The reaction mixture was stirred at 50 °C for 0.5 h. A solution of 4-(5-bromopentylthio)-7-(trifluoromethyl)quinoline (2.00 g, 5.29 mmol) in 5 mL of DMF was added via syringe. The organic mixture was stirred under nitrogen at 85 °C for 3 h. After cooling the mixture to room temperature DMF was evaporated. 50 mL of H₂O were added to the reaction mixture which was extracted with EtOAc (2 x 100 mL). The organic layer was washed with water (3 x 50 mL) and brine (2 x 50 mL), dried over MgSO₄, filtered and evaporated. The crude product was purified by column chromatography on silica using as eluent CH₂Cl₂:MeOH = 95:5. After evaporation and drying a white solid 4-(5-(2-methoxy-4-((piperidin-1-yl)methyl)phenoxy)pentylthio)-7-(trifluoromethyl)quinoline was obtained (1.32 g). The product was dissolved in ethanol (5 mL) and this solution was stirred at RT. Aqueous HCl (1 M, 70 mL) was added via syringe at RT for 1 h. The solution was evaporated to dryness and triturated with EtOAc. After filtration and drying 4-(5-(2-methoxy-4-((piperidin-1-yl)methyl)phenoxy)pentylthio)-7-(trifluoromethyl)quinoline dihydrochloride 7 (1.36 g, 43 % yield) was obtained as a yellow solid.

The structure of compound example 7 is presented below: 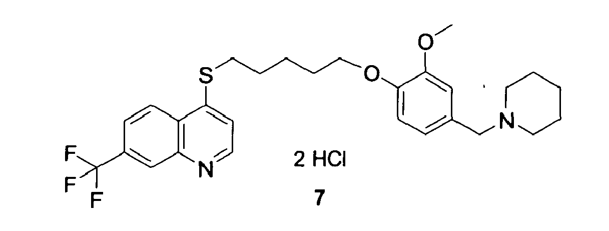 **MW:** 591.56; **Yield:** 43 %; Yellow Solid; **Mp** (°C): 182.6.
**R*_{f}***: 0.30 (MeOH: CH₂Cl₂ = 10:90).
**¹H-NMR** (CDCl₃, δ): 1.50-2.08 (m, 12H, 6xCH₂), 3.10 (s broad, 4H, 2xCH₂), 3.31-3.42 (m, 2H, CH₂), 3.88 (s, 3H, OMe), 4.11 (t, 2H, *J* = 5.7 Hz, CH₂), 4.19 (s, 2H, CH₂), 7.05-7.08 (m, 1H, ArH), 7.16 (s, 2H, ArH), 7.58 (d, 1H, *J* = 4.8 Hz, ArH), 7.85 (d, 1H, *J* = 8.8 Hz, ArH), 8.29 (s, 1H, ArH), 8.36 (d, 1H, *J* = 8.8 Hz, ArH), 8.77 (d, 1H, *J =* 4.8 Hz, ArH).
**MS-ESI** m/z (% rel. Int.): 519.1 ([MH]⁺, 100), 298.1 (40).
**HPLC:** Method A, detection UV 254 nm, RT = 5.10 min, peak area 99.9 %.

### Example 8: 4-(5-(4-(morpholinomethyl)phenoxy)pentylthio)-7-(trifluoromethyl)quinoline dihydrochloride 8.

### 4-(Morpholinomethyl)phenol.

To a stirred solution of 4-hydroxybenzaldehyde (6.11 g, 0.05 mmol) in morpholine (8.67 mL, 0.10 mmol) was added *via* syringe formic acid (4.9 mL, 0.13 mmol). The mixture was stirred for 2 h at 115 °C and after cooling poured in water (300 mL). The solution was acidified by a solution of aqueous HCl (1 M, 40 mL) and extracted with diethyl ether (4 x 100 mL). The aqueous solution was basified with a solution of NH₄OH (20% water, 14 mL). The white precipitate was filtered off and washed well with water and hexane. After drying 4-(morpholinomethyl)phenol (7.74 g, 80 % yield) was obtained as a white solid. 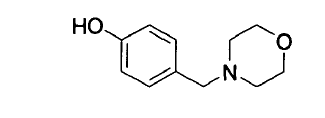 **MW:** 193.24; **Yield:** 80 %; White Solid; **Mp** (°C): 109.4 (dec).
**¹H-NMR** (CDCl₃, δ): 2.50 (s broad, 4 H, CH₂), 3.45 (s, 2H, N-CH₂), 3.73 (m, 4H, N-CH₂), 6.66 (d, 2H, *J* = 8.4 Hz, Ar-H), 7.12 (d, 2H, *J* = 8.4 Hz, Ar-H), OH not seen.

### 4-(5-(4-(Morpholinomethyl)phenoxy)pentylthio)-7-(trifluoromethyl)quinoline dihydrochloride 8.

4-(Morpholinomethyl)phenol (1.07 g, 5.55 mmol) was dissolved in DMF (10 mL) and cesium carbonate (1.81 g, 5.55 mmol) was added under a nitrogen atmosphere. The reaction mixture was stirred at 50 °C for 0.5 h. A solution of 4-(5-bromopentylthio)-7-(trifluoromethyl)quinoline (2.00 g, 5.29 mmol) in 5 mL of DMF was added *via* syringe. The organic mixture was stirred under nitrogen at 85 °C for 3 h. After cooling the mixture to room temperature DMF was evaporated. 50 mL of H₂O were added to the reaction mixture which was extracted with EtOAc (2 x 100 mL). The organic layer was washed with water (3 x 50 mL) and brine (2 x 50 mL), dried over MgSO₄, filtered and evaporated. The crude product was purified by column chromatography (SiO₂; eluent CH₂Cl₂:MeOH = 95:5). After evaporation and drying a white solid 4-(5-(4-(morpholinomethyl)phenoxy)pentylthio)-7-(trifluoromethyl)quinoline was obtained (1.86 g, 72 % yield).
The product was dissolved in ethanol (5 mL) and this solution was stirred at RT. Aqueous HCl (1 M, 70 mL) was added *via* syringe at RT for 1 h. The solution was evaporated to dryness and triturated with EtOAc. After filtration and drying 4-(5-(4-(morpholinomethyl)phenoxy)pentylthio)-7-(trifluoromethyl)quinoline dihydrochloride **8** was obtained (1.83 g, 61 % yield) as a white solid.

The structure of compound example 8 is presented below: 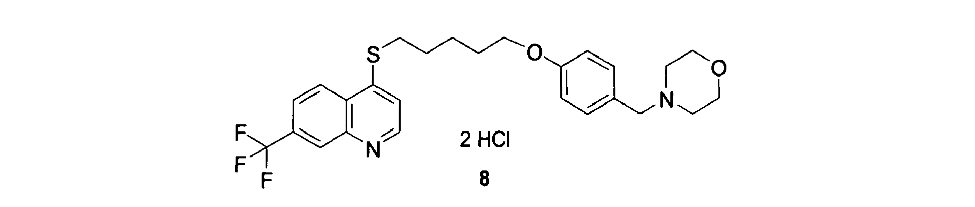 **MW:** 563.61; **Yield:** 61 %; White Solid; **Mp** (°C): 220.5.
**R*_{f}*:** 0.30 (MeOH:CH₂Cl₂ = 10:90).
**¹H-NMR** (CDCl₃, δ): 1.70-1.97 (m, 6H, 3xCH₂), 3.04 (s broad, 4H, 2xCH₂), 3.21-3.30 (m, 2H, CH₂), 3.83 (s broad, 4H, 2xCH₂), 3.95-4.15 (m, 4H, 2xCH₂), 6.97 (d, 2H, *J* = 7.9 Hz, ArH), 7.38 (d, 2H, *J=* 8.1 Hz, ArH), 7.53 (d, 1H, *J=* 4.7 Hz, ArH), 7.82 (d, 1H, *J=* 8.8 Hz, ArH), 8.26 (s, 1H, ArH), 8.35 (d, 1H, *J =* 8.7 Hz, ArH), 8.73 (d, 1H, *J =* 4.3 Hz, ArH).
**MS-ESI** m/z (% rel. Int.): 491.0 ([MH]⁺, 15), 404.0 (65), 298.0 (80), 230.0 (30), 106.9 (100).
**HPLC:** Method A, detection UV 254 nm, RT = 4.87 min, peak area 99.9 %

### Example 9: 4-(5-(2-methoxy-4-(morpholinomethyl)phenoxy)pentylthio)-7-(trifluoromethyl)quinoline dihydrochloride 9.

### 2-Methoxy-4-(morpholinomethyl)phenol.

To a stirred solution of 4-hydroxy-3-methoxybenzaldehyde (7.61 g, 0.05 mmol) in morpholine (8.67 mL, 0.10 mmol) was added *via* syringe formic acid (4.9 mL, 0.13 mmol). The mixture was stirred for 2 h at 115 °C and after cooling poured in water (300 mL). The solution was acidified by a solution of aqueous HCl (1 M, 40 mL), and extracted with diethyl ether (4 x 100 mL). The aqueous solution was basified with a solution of NH₄OH (20% water, 14 mL). The white precipitate was filtered off and washed well with water and hexane. After drying 2-methoxy-4-(morpholinomethyl)phenol (10.77 g, 96.5 % yield) was obtained as a brown oil. 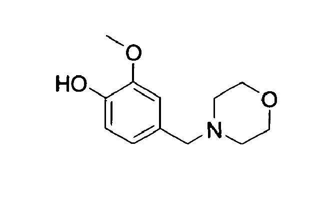 **MW:** 223.27; **Yield:** 96.5 %; Brown Oil.
**¹H-NMR** (CDCl₃, δ): 2.59 (s broad, 4H, CH₂), 3.69 (s, 2H, N-CH₂), 3.76 (t, 4H, *J* = 4.6 Hz, N-CH₂), 3.88 (s, 3H, OMe), 6.61 (dd, 2H, *J=* 1.1 Hz, *J=* 7.4 Hz, Ar-H), 6.76 (t, 1H, *J* = 7.5 Hz, Ar-H), 6.82 (dd, 1H, *J =* 1.1 Hz, *J =* 7.4 Hz, Ar-H), OH not seen.

### 4-(5-(2-Methoxy-4-(morpholinomethyl)phenoxy)pentylthio)-7-(trifluoromethyl)quinoline dihydrochloride 9.

2-Methoxy-4-(morpholinomethyl)phenol (1.24 g, 5.55 mmol) was dissolved in DMF (10 mL) and cesium carbonate (1.81 g, 5.55 mmol) was added under a nitrogen atmosphere. The reaction mixture was stirred at 50 °C for 0.5 h. A solution of 4-(5-bromopentylthio)-7-(trifluoromethyl)quinoline (2.00 g, 5.29 mmol) in 5 mL of DMF was added *via* syringe. The organic mixture was stirred under nitrogen at 85 °C for 3 h. After cooling the mixture to room temperature DMF was evaporated. 50 mL of H₂O were added to the reaction mixture which was extracted with EtOAc (2 x 100 mL). The organic layer was washed with water (3 x 50 mL) and brine (2 x 50 mL), dried over MgSO₄, filtered and evaporated. The crude product was purified by column chromatography on silica using as eluent CH₂Cl₂:MeOH = 95:5. After evaporation and drying a white solid 4-(5-(2-methoxy-4-(morpholinomethyl)phenoxy)pentylthio)-7-(trifluoromethyl)quinoline (1.83 g, 67 % yield). The product was dissolved in ethanol (5 mL) and this solution was stirred at RT. Aqueous HCl (1 M, 70 mL) was added via syringe at RT for 1 h. The solution was evaporated to dryness and triturated with EtOAc. After filtration and drying 4-(5-(2-methoxy-4-(morpholinomethyl)phenoxy)pentylthio)-7-(trifluoromethyl)quinoline dihydrochloride **9** was obtained (1.76 g, 56 % yield) as a white solid.

The structure of compound example 9 is presented below: 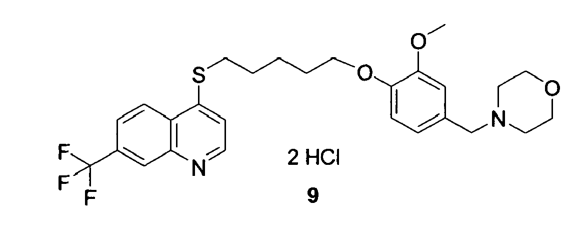 **MW:** 593.53; **Yield:** 56 %; White Solid; **Mp** (°C): 200.5
**R*_{f}*:** 0.30 (MeOH: CH₂Cl₂ = 10:90).
**¹H-NMR** (CDCl₃, δ): 1.70-2.01 (m, 6H, 3xCH₂), 2.98 (s broad, 4H, 2xCH₂), 3.29-3.31 (m, 2H, S-CH₂), 3.77 (s broad, 4H, 2xCH₂), 3.85 (s, 3H, OMe), 4.05-4.09 (m, 4H, CH₂), 7.01-7.15 (m, 3H, ArH), 7.56 (d, 1H, *J=* 4.9 Hz, ArH), 7.82 (d, 1H, *J=* 8.8 Hz, ArH), 8.27 (s, 1H, ArH), 8.36 (d, 1H, *J* = 8.8 Hz, ArH), 8.74 (d, 1H, *J =* 4.5 Hz, ArH).
**MS-ESI** m/z (% rel. Int.): 521.0 ([MH]⁺, 100), 434.0 (55), 298.1 (50), 230.0 (35), 137.0 (40).
**HPLC:** Method A, detection UV 254 nm, RT = 4.90 min, peak area 98.6 %.

### General Procedure for examples 10 & 11: Method C.

To a solution of aldehyde (5 mmol) in MeOH was added piperidine (426 mg, 5 mmol). The reaction mixture was stirred for 2 h at RT, treated with NaCNBH₃ (377 mg, 6 mmol) and stirred an additional 16 h at RT. All the volatiles were evaporated and the reaction mixture was partitioned between EtOAc and brine. The aqueous phase was extracted twice with EtOAc. The combined organic fractions were dried over Na₂SO₄, filtrated and evaporated to give a crude solid that was purified by column chromatography using EtOAc to give the title compound.

### Example 10: 4-(5-(2-chloro-4-((piperidin-1-yl)methyl)phenoxy)pentylthio)-7-(trifluoromethyl)quinoline dihydrochloride 10.

### 2-Chloro-4-((piperidin-1-yl)methyl)phenol.

The compound was prepared according to method C using 3-chloro-4-hydroxybenzaldehyde (782 mg, 5.0 mmol). 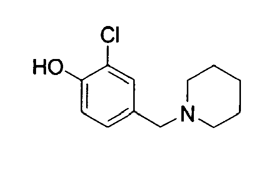 **MW:** 225.7; **Yield:** 46 %; Brown Oil.
*R_{f}*: 0.3 (EtOAc = 100%).
**¹H-NMR** (CDCl₃, δ): 1.40-1.50 (m, 2H, CH₂), 1.55-1.65 (m, 4H, 2×CH₂), 2.35-2.50 (m, 4H, 2×CH₂), 3.40 (s, 2H, N-CH₂), 4.40 (s broad, 1H, O-H), 6.83 (d, 1H, *J =* 8.3 Hz, Ar-H), 7.07 (dd, 1H, *J =* 2.0 Hz, 8.3 Hz, Ar-H), 7.27 (d, 1H, *J =* 2.0 Hz, Ar-H).
**¹³C-NMR** (CDCl₃,δ): 24.2, 25.6 (2×C), 54.3 (2×C), 62.7, 116.0, 119.9, 129.4, 129.9, 130.6, 150.8.
**MS-ESI** *m*/*z* (rel. int.): 226.1 ([MH]⁺, 100).
**HPLC:** Method A, detection UV 254 nm, RT = 3.67 min, peak area 95.0 %.

### 4-(5-(2-Chloro-4-((piperidin-1-yl)methyl)phenoxy)pentylthio)-7-(trifluoromethyl)quinoline dihydrochloride 10.

To a solution of anhydrous of 2-chloro-4-((piperidin-1-yl)methyl)phenol (200 mg, 0.886 mmol) in anhydrous DMF (10 mL) stirred for 10 min. was added 4-(5-bromopentylthio)-7-(trifluoromethyl)quinoline (0.318 g, 0.847 mmol) and the reaction mixture was stirred for 1 h at 80 °C. The volatiles were evaporated and the reaction mixture was partitioned between EtOAc and brine. The organic layer was separated and the aqueous phase was extracted with EtOAc. The combined organic fractions were dried over Na₂SO₄, filtered and the crude product was purified by column chromatography (SiO₂, MeOH:EtOAc = 20:80) to obtain 4-(5-(2-chloro-4-((piperidin-1-yl)methyl)phenoxy)pentylthio)-7-(trifluoromethyl)quinoline. This white solid (168 mg) was treated with an ice-cold solution of HCl (1 N) in *i*-PrOH (12.8 mL). The volatiles were evaporated to obtain 4-(5-(2-chloro-4-((piperidin-1-yl)methyl)phenoxy)pentylthio)-7-(trifluoromethyl)quinoline dihydrochloride 10 as white solid (190 mg, 36 % yield).

The structure of compound example 10 is presented below: 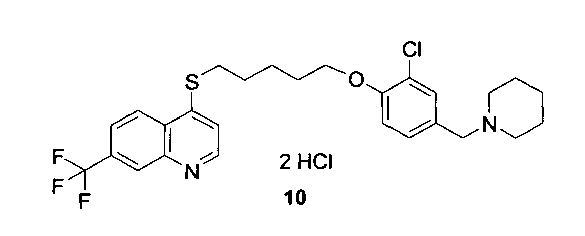 **MW:** 596.0; **Yield:** 36 %; White Solid; **Mp** (°C): 78.8.
***R_{f}:*** 0.3 (EtOAc = 100%), free base.
**¹H-NMR** (CD₃OD, δ): 1.75-2.15 (m, 12H, CH₂), 2.94 (t, 2H, N-CH), 3.42 (d, 2H, *J* = 12.4 Hz, S-CH₂), 3.55 (t, 2H, *J =* 7.2 Hz, N-CH), 4.16 (t, 2H, *J=* 5.7 Hz, O-CH₂), 4.22 (s, 2H, N-CH₂), 7.17 (d, 1H, *J =* 7.2 Hz, Ar-H), 7.43 (d, 1H, J = 8.4 Hz, Ar-H), 7.58 (s, 1H, Ar-H), 8.04 (d, 1H, *J =* 6.3 Hz, Ar-H), 8.16 (d, 1H, J = 9.0 Hz, Ar-H), 8.47 (s, 1H, Ar-H).
**MS-ESI** *m*/*z* (rel. int.): 523.0 ([MH]⁺, 100).
**HPLC:** Method A, detection UV 254 nm, RT = 5.18 min, peak area 98.3 %.

### Example 11: 4-(5-(2-nitro-4-((piperidin-1-yl)methyl)phenoxy)pentylthio)-7-(trifluoromethyl)quinoline dihydrochloride 11.

### 2-Nitro-4-((piperidin-1-yl)methyl)phenol.

The compound was prepared according to method C using 4-hydroxy-3-nitrobenzaldehyde (782 mg, 5.0 mmol). 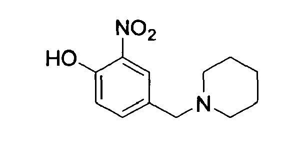 **MW:** 236.3; Yield: 37 %; Red Solid. **Mp** (°C): 125.8.
***R_{f}*:** 0.3 (EtOAc = 100%).
**¹H-NMR** (CDCl₃, δ): 1.45-1.51 (m, 2H, CH₂), 1.55-1.65 (m, 4H, 2×CH₂), 1.90-1.95 (m, 2H, N-CH₂), 2.48-2.55 (m, 4H, N-CH₂), 3.55 (s, 2H, N-CH₂), 7.15 (d, 1H, *J =* 8.6 Hz, Ar-H), 7.64 (dd, 1H, *J =* 2.1 Hz, *J =* 8.6 Hz, ArH), 8.05 (d, 1 H, *J =* 2.1 Hz, ArH).
**MS-ESI** *m*/*z* (rel. int.): 237.1 ([MH]⁺, 100).
**HPLC:** Method A, detection UV 254 nm, RT = 3.68 min, peak area 95.0 %.

### 4-(5-(2-Nitro-4-((piperidin-1-yl)methyl)phenoxy)pentylthio)-7-(trifluoromethyl)quinoline dihydrochloride 11.

To a solution of anhydrous of 2-nitro-4-((piperidin-1-yl)methyl)phenol (200 mg, 0.846 mmol) in anhydrous DMF (10 mL) stirred for 10 min was added 4-(5-bromopentylthio)-7-(trifluoromethyl)quinoline (0.304 g, 0.804 mmol) and the reaction mixture was stirred for 1 h at 80 °C. The volatiles were evaporated and the reaction mixture was partitioned between EtOAc and brine. The organic layer was separated and the aqueous phase was extracted with EtOAc. The organic fractions were combined, dried over Na₂SO₄, filtered and the crude product was purified by column chromatography (SiO₂, MeOH:EtOAc = 20:80) to obtain 4-[5-(2-nitro-4-piperidin-1-ylmethyl-phenoxy)-pentylsulfanyl]-7-trifluoromethylquinoline as a white solid (273 mg) that was treated with a ice-cold solution of HCl (1 N) in iPrOH (20 mL). The volatiles were evaporated to obtain 4-(5-(2-nitro-4-((piperidin-1-yl)methyl)phenoxy)pentylthio)-7-(trifluoromethyl)quinoline dihydrochloride 11 as a white solid (310 mg, 60 % yield).

The structure of compound example 11 is presented below: 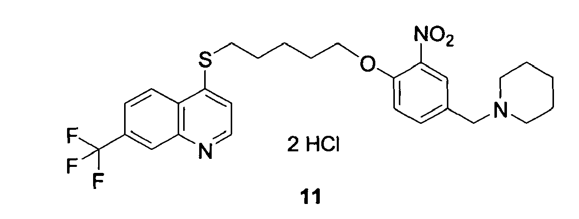 **MW:** 606.5; **Yield:** 60 %; White Solid; **Mp** (°C): 115.2
***R_{f}***: 0.3 (EtOAc = 100%), free base.
**¹H-NMR** (CD₃OD, δ): 1.46-1.60 (m, 1H, CH₂), 1.70-2.10 (m, 11H, CH₂), 2.97 (t, 2H, *J =* 11.6 Hz, N-CH), 3.56 (t, 2H, *J* = 7.1 Hz, 2xN-CH), 4.26 (t, 2H, 2xN-CH), 4.93 (s, 2H, O-CH₂), 7.40 (d, 1H, *J* = 8.2, Ar-H), 7.80 (d, 1H, *J* = 8.7 Hz, Ar-H), 8.00-8.07 (m, 2H, Ar-H), 8.16 (d, 1H, *J =* 8.9 Hz, Ar-H), 8.47 (s, 1H, Ar-H), 8.69 (d, 1H, *J* = 8.9, Ar-H), 9.00 (s, 1H, *J* = 6.3 Hz).
**MS-ESI** *m*/*z* (rel. int.): 534.0 ([MH]⁺, 100).
**HPLC:** Method A, detection UV 254 nm, RT = 5.03 min, peak area 99.9 %.

### Example 12: (4-(5-(7-(trifluoromethyl)quinolin-4-ylthio)pentyloxy)phenyl)methanol hydrochloride 12.

To a solution of 4-hydroxybenzaldehyde (200 mg, 1.6 mmol) in DMF was added Cs₂CO₃ (521 mg 1.6 mmol). The reaction mixture was stirred 0.33 h at 25 °C. 4-(5-bromopentylthio)-7-(trifluoromethyl)quinoline (574 mg, 1.52 mmol) was added and the reaction mixture was heated at 80 °C for 2 h. All the solvents were evaporated and the reaction was partitioned between EtOAc and brine. The desired product was extracted with EtOAc and the combined fractions were dried over Na₂SO₄, filtered to give an oily residue that was purified using column chromatography (SiO₂, EtOAc: cyclohexane = 50:50) to give (4-(5-(7-(trifluoromethyl)quinolin-4-ylthio)pentyloxy)phenyl)methanol as a white solid (449 mg, 70% yield, Mp = 98.3 °C). A solution of (4-(5-(7-(trifluoromethyl)quinolin-4-ylthio)pentyloxy)phenyl)methanol (200 mg, 0.474 mmol) in MeOH was prepared, stirred at 0-4 °C and a solution of HCl (0.1 N) in iPrOH (9.5 mL) was added. All the volatiles were evaporated to give (4-(5-(7-(trifluoromethyl)quinolin-4-ylthio)pentyloxy)phenyl)methanol hydrochloride 12 as a white solid (217 mg, 0.474 mmol).

The structure of compound example 12 is presented below: 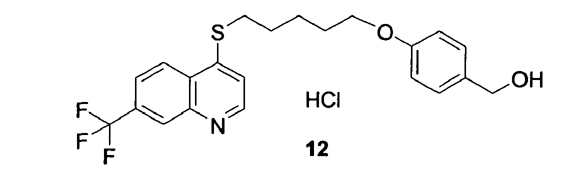 **MW:** 421.5; **Yield:** 70 %; White Solid; **Mp** (°C): 141.7
***R_{f}*:** 0.3 (EtOAc:cyclohexane = 1:1), free base.
**¹H-NMR** (CD₃OD, δ): 1.71-1.95 (m, 4H, CH₂), 1.95-2.18 (m, 2H, CH₂), 3.52 (t, 2H, *J =* 7.2 Hz, S-CH₂), 4.00 (t, 2H*, J* = 5.8 Hz, OCH₂), 4.50 (s, 2H, OCH₂), 6.84(d, 2H, *J* = 8.5 Hz, Ar-H), 7.22 (d, 2H, *J* = 8.3 Hz, Ar-H), 8.00 (d, 1H, *J* = 6.3 Hz, Ar-H), 8.15 (d, 1H, *J* = 9.0 Hz, Ar-H), 8.45 (s, 1H, Ar-H), 8.66 (d, 1H, *J =* 8.9 Hz, Ar-H), 8.97 (d, 1H, *J =* 6.3 Hz, Ar-H), OH not seen.
**MS-ESI** *m*/*z* (rel. int.): 422.2 ([MH]⁺, 5), 404.1 (100).
**HPLC:** Method A, detection UV 254 nm, RT = 5.48 min, peak area 95.3 %.

### Example 13: 2-(5-(7-(trifluoromethyl)quinolin-4-ylthio)pentyloxy)-5-((piperidin-1-yl)methyl)benzenamine trihydrochloride 13.

### tert-Butyl 2-hydroxy-5-((piperidin-1-yl)methyl)phenylcarbamate.

To a solution of 2-nitro-4-((piperidin-1-yl)methyl)phenol (503 mg, 2.13 mmol) in MeOH degassed with nitrogen was added palladium on carbon 10 % [w/w] (150 mg) and the mixture was stirred in a Parr apparatus with H₂ at 4 atm for 2 h. Di-*tert*-butyl-dicarbonate (511, mg, 2.34 mmol) was added and the the reaction mixture was stirred at 25 °C for 16 h. The reaction mixture was filtered over celite, evaporated and the product purified by column chromatography using MeOH 20 % [v/v] in EtOAc to obtain *tert*-butyl 2-hydroxy-5-((piperidin-1-yl)methyl)phenylcarbamate (192 mg, 29 % yield) as a pale red solid. 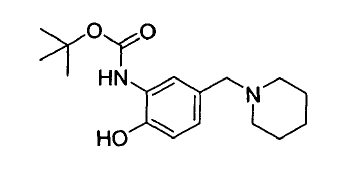 **MW:** 306.4; **Yield:** 29 %; Pale Red Solid; **Mp** (°C): 89.1
***R_{f}*:** 0.3 (MeOH: EtOAc, 20:80).
**¹H-NMR** (CDCl₃, δ): 1.40-1.50 (m, 2H, CH₂), 1.51 (s, 9H, C(CH₃)₃), 1.60-1.68 (m, 4H, CH₂), 1.40-1.60 (m, 4H, CH₂), 3.41 (s, 2H, CH₂), 6.44 (d, 1H, *J* = 8.1Hz, Ar-H), 6.72 (dd, 1H, J = 1.9 Hz, *J =* 8.1 Hz, Ar-H), 6.98 (s, 1H, NH), 7.45 (bs, 1H, OH), 7.48 (d, 1H, *J =* 1.9 Hz, Ar-H).
**MS-ESI** *m*/*z* (rel. int.): 307.1 ([MH]⁺, 20), 166.0 (100).
**HPLC:** Method A, detection UV 254 nm, RT = 4.38 min, peak area 96.9 %.
*tert*-Butyl 2-(5-(7-(trifluoromethyl)quinolin-4-ylthio)pentyloxy)-5-((piperidin-1-yl)methyl)phenylcarbamate.

To a solution of *tert*-butyl 2-hydroxy-5-((piperidin-1-yl)methyl)phenylcarbamate (165 mg, 0.54 mmol) in DMF (10 mL) was added Cs₂CO₃ (176 mg) and the reaction was stirred for 10 min. 4-(5-Bromopentylthio)-7-(trifluoromethyl)quinoline (176 mg, 0.54 mmol) was added and the reaction mixture was heated at 80 °C for 2 h. All the volatiles were evaporated at 70°C and the residue obtained was partitioned between EtOAc and H₂O. The desired product was extracted with EtOAc, dried over Na₂SO₄, evaporated to give a residue that was purified by silica gel chromatography using 10 % [v/v] MeOH in EtOAc to obtain *tert-*butyl 2-(5-(7-(trifluoromethyl)quinolin-4-ylthio)pentyloxy)-5-((piperidin-1-yl)methyl)phenylcarbamate as a pale brown solid. (213 mg, 69 % yield). 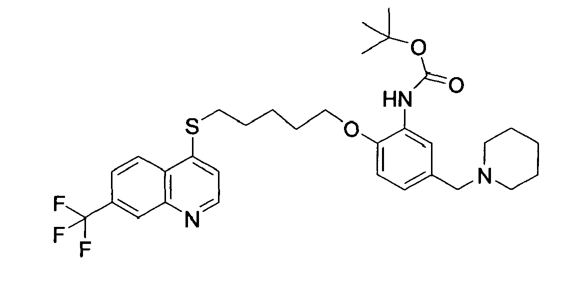 **MW:** 603.7; **Yield:** 69 %; Pale Brown Solid.
***R_{f}:*** 0.3 (MeOH: EtOAc = 10:90).
**¹H-NMR** (CDCl₃, δ): 1.35-1.47 (m, 2H, CH₂), 1.52 (s, 9H, C(CH₃)₃), 1.55-1.65 (m, 6H, CH₂), 1.70-1.80 (m, 1H, CH), 1.82-1.98 (m, 4H, CH₂), 2.30-2.45 (m, 4H, 2xCH₂), 3.08-3.20 (m, 2H, S-CH₂), 3.45 (t, 2H, *J* = 6.7 Hz, NCH₂), 3.87 (t, 1H, *J* = 7.0 Hz, OC-H), 4.04 (t, 1H, *J* = 6.4 Hz, OC-H), 6.75-7.15 (m, 3H, Ar-H), 7.27 (s, 1H, Ar-H), 7.72 (d, 1H, *J =* 8.8 Hz, Ar-H), 7.97 (s, 1H, NH), 8.23 (t, 1H, *J =* 5.5 Hz, Ar-H), 8.36 (s, 1H, Ar-H), 8.79 (d, 1H, Ar-H).
**MS-ESI** *m*/*z* (rel. int.): 604.2 ([MH]⁺, 100);
**HPLC:** Method A, detection UV 254 nm, RT = 5.43 min, peak area 69.8 %.

### 2-(5-(7-(Trifluoromethyl)guinolin-4-ylthio)pentyloxy)-5-((piperidin-1-yl)methyl)benzenamine trihydrochloride 13.

To a solution *tert-*butyl 2-(5-(7-(trifluoromethyl)quinolin-4-ylthio)pentyloxy)-5-((piperidin-1-yl)methyl)phenylcarbamate (222 mg, 0.37 mmol) in MeOH (5 mL) was added in concentrated HCl (12 N, 1 mL, 12 mmol) stirred for 1 h and evaporated to dryness. The resulting solid was partitioned between EtOAc (25 mL) and aqueous saturated Na₂CO₃ (25 mL). The organic layer was separated from the aqueous phase which was extracted with EtOAc (3 x 50 mL). The organic layers were combined, evaporated to give a solid that was purified by silica gel chromatography using 30 % [v/v] MeOH in EtOAc to afford 2-(5-(7-(trifluoromethyl)quinolin-4-ylthio)pentyloxy)-5-((piperidin-1-yl)methyl)benzenamine (34 mg). Conversion to the hydrochloride salt was accomplished using a solution of HCl (0.1 N) in iPrOH (0.22 mmol, 2.2 mL) to obtain after evaporation of the volatiles 2-(5-(7-(trifluoromethyl)quinolin-4-ylthio)pentyloxy)-5-((piperidin-1-yl)methyl)benzenamine trihydrochloride 13 (51 mg, 22 % yield) as a white solid.

The structure of compound example 13 is presented below: 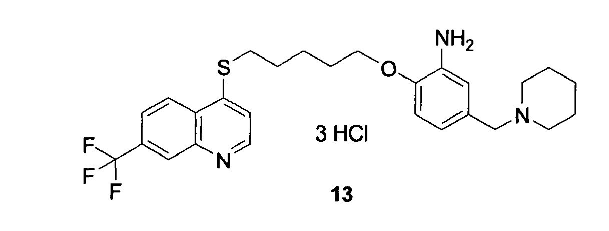 **MW:** 613.01; **Yield:** 22 %; White Solid; **Mp** (°C): 71.4.
***R_{f}***: 0.3 (MeOH:EtOAc = 20:80), free base.
**¹H-NMR** (CD₃OD, δ) : 1.40-1.60 (m, 1H, CH₂), 172-2.10 (11H, CH₂), 2.97 (t, 2H, *J* = 11.0, S-CH₂), 3.30 (s, 4H, N-CH₂), 3.48 (d, 2H, *J =* 12.0, N-CH₂), 3.54 (t, 2H, *J =* 7.1 Hz, OCH₂), 4.20 (m, 4H, N-CH₂), 7.31 (d, 1H, *J =* 8.4 Hz, Ar-H), 7.60 (dd, 1H, 8.5 Hz, *J =* 1.7 Hz, Ar-H), 7.66 (s, 1H, N-CH₂), 8.03 (d, 1H, *J=* 6.1 Hz, Ar-H), 8.13 (d, 1H, *J=* 8.9 Hz, Ar-H), 8.46 (s, 1H, Ar-H), 8.65 (d, 1H, *J* = 8.8 Hz, Ar-H), 9.00 (d, 1H, *J* = 6.1 Hz, Ar-H).
**MS-ESI** *m*/*z* (rel. int.): 307.1 ([MH]⁺, 20), 166.0 (100).
**HPLC:** Method A, detection UV 254 nm, RT = 4.75 min, peak area 99 %.

### Example 14: (S)-ethyl 3-(4-(5-(7-(trifluoromethyl)quinolin-4-ylthio)pentyloxy)phenyl)-2-acetamidopropanoate hydrochloride 14.

*N*-Acetyl-L-tyrosine ethyl ester (0.72 g, 2.6 mmol), Cs₂CO₃ (0.75 g, 2.8 mmol), and anhydrous DMF (10 mL) were charged in a 50 ml round-bottomed flask equipped with a magnetic stirrer under inert atmosphere. 4-(5-Bromopentylthio)-7-(trifluoromethyl)quinoline (1.00 , 2.6 mmol) was added and the reaction mixture was stirred for 2 h at 90 °C. After evaporation of DMF the reaction mixture was poured in 50 mL of H₂O and extracted with EtOAc (2 x 100 mL). The organic layer was washed with brine (2 x 20 mL), dried over MgSO₄, filtered and evaporated to dryness. The crude compound was purified by column chromatography (SiO₂, CH₂Cl₂:EtOAc = 5:5) to give after evaporation (*S*)-ethyl 3-(4-(5-(7-(trifluoromethyl)quinolin-4-ylthio)pentyloxy)phenyl)-2-acetamidopropanoate as a solid (680 mg, 50 % yield). The compound was dissolved in MeOH (3 mL) and a solution of HCl 1M in MeOH (1.3 mL, 1.3 mmol) was added. After evaporation of MeOH the product was dried under vacuum to yield to (*S*)-ethyl 3-(4-(5-(7-(trifluoromethyl)quinolin-4-ylthio)pentyloxy)phenyl)-2-acetamidopropanoate hydrochloride **14** (750 mg, 50 % yield) as a beige powder.

The structure of compound example 14 is presented below: 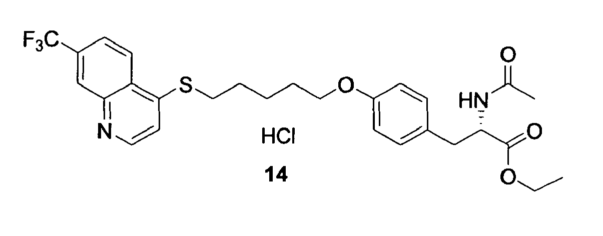 **MW:** 585.08; **Yield:** 50 %; Beige Powder, **Mp** (°C): 155.9.
***R_{f}***: 0.25 (CH₂Cl₂:EtOAc= 5:5), free base.
**¹H-NMR** (CD₃OD, δ): 1.14-1.23 (m, 3H, CH₃), 1.79-2.02 (m, 9H, 3xCH₂ & CH₃), 2.82-3.08 (m, 2H, S-CH₂), 3.49-3.55 (m, 2H, Ar-CH₂), 3.96-4.02 (m, 2H, O-CH₂), 4.08-4.17 (m, 2H, O-CH₂), 4.53-4.59 (m, 1H, N-CH), 6.79-6.84 (m, 2H, Ar-H), 7.07-7.11 (m, 2H, Ar-H) 7.99-8.03 (m, 1H, Ar-H), 8.14-8.17 (m, 1H, Ar-H), 8.46 (s, 1H, Ar-H), 8.64-8.70 (m, 1H, Ar-H), 8.95-9.01 (m, 1H, Ar-H).
**MS-ESI** *m*/*z* (rel. int.): 549.1 ([MH]⁺, 100).
**HPLC:** Method A, detection UV 254 nm, RT = 5.8 min, peak area 98.0 %.

### Example 15: 4-(5-(6-methylpyeridin-3-yloxy)pentylthio)-7-(trifluoromethyl)quinoline dihydrochloride 15.

3-Hydroxy-6-methylpyridine (72 mg, 0.66 mmol), Cs₂CO₃ (215 mg, 0.66 mmol), NaI (40 mg, 0.66 mmol) and anhydrous DMF (3 mL) were charged in a 25 ml round-bottomed flask equipped with a magnetic stirrer under inert atmosphere. 4-(5-Bromopentylthio)-7-(trifluoromethyl)quinoline (250 mg, 0.66 mmol) was added and the reaction mixture was stirred for 3 h at 90 °C. After evaporation of DMF, the reaction mixture was poured in 50 mL of H₂O and extracted with EtOAc (2 x 100 mL). The organic layer was washed with brine (2 x 20 mL), dried over MgSO₄, filtered and evaporated to dryness. The crude compound was purified by column chromatography (SiO₂; EtOAc:MeOH = 99:1) to give after evaporation and drying a solid (190 mg, 71 % yield). The solid was dissolved in MeOH (3 mL) and a solution of HCl 1M in MeOH (1.0 mL, 1.0 mmol) was added. After removal of MeOH, the hydrochloride compound was dried under vacuum to yield to 4-(5-(6-methylpyridin-3-yloxy)pentylthio)-7-(trifluoromethyl)quinoline dihydrochloride **15** (180 mg, 57 % yield) as a pale yellow powder. 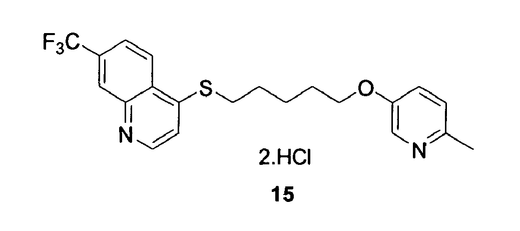 **MW:** 479.39; **Yield:** 57%; Pale Yellow Powder, **Mp** (°C): 188.2.
***R_{f}:*** 0.60 (EtOAc:MeOH = 9:1).
**¹H-NMR** (CD₃OD, δ): 1.80-1.96 (m, 2H, CH₂), 1.99-2.06 (m, 4H, 2xCH₂), 2.72 (s, 3H, CH₃), 3.55 (t, 2H, *J=* 6.7 Hz, S-CH₂), 4.27 (t, 2H, *J=* 5.5 Hz, O-CH₂), 7.83 (d, 1H, *J=* 8.7 Hz, Ar-H), 8.05 (d, 1H, *J =* 5.5 Hz, Ar-H) 8.10-8.18 (m, 2H, Ar-H), 8.43 (s broad, 1H, Ar-H), 8.50 (s broad, 1H, Ar-H), 8.66 (d, 1H, *J =* 8.8 Hz, Ar-H), 9.02 (d, 1H, *J =* 5.4 Hz, Ar-H).
**MS-ESI** *m*/*z* (rel. int.): 407.1 ([MH]⁺, 100).
**HPLC:** Method A, detection UV 254 nm, RT = 4.6 min, peak area 96.1 %

### Example 16:4-(5-(2-methoxy-6-nitro-4-((piperidin-1-yl)methyl)phenoxy)pentylthio)-7-(trifluoromethyl)quinoline 16.

### 2-Methoxy-6-nitro-4-((piperidin-1-yl)methyl)phenol.

To a solution of 4-hydroxy-3-methoxy-5-nitrobenzaldehyde (3.13 g, 15.90 mmol) in MeOH (30 mL) was added piperidine (1.6 mL, 15.9 mmol) in a 100 mL round-bottomed flask equipped with a magnetic stirrer. The reaction mixture was stirred for 2 h at RT. NaCNBH₃ (1.2 g, 9.5 mmol) was added and the reaction mixture was stirred at RT for 24 h. The precipitate was filtered off and MeOH was evaporated at 40 °C to dryness. The crude compound was purified by column chromatography (SiO₂; eluent CH₂Cl₂:MeOH = 95:5 to 90:10) to give after evaporation 2-methoxy-6-nitro-4-((piperidin-1-yl)methyl)phenol (1.60 g, 40 % yield) as an orange solid. 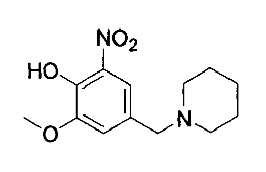 **MW:** 266.29; **Yield:** 40 %; Orange Solid; Mp (°C) = 139.9
***R_{f}*:** 0.30 (CH₂Cl₂:MeOH = 9:1).
**¹H-NMR** (CD₃OD, δ): 1.62-1.70 (m, 2H, CH₂), 1.78-1.88 (m, 4H, CH₂), 3.07 (s broad, 4H, NCH₂), 3.86 (s, 3H, O-CH₃), 4.02 (s, 2H, N-CH₂), 7.08 (d, 1H, *J=* 1.8 Hz, Ar-H), 7.65 (d, 1H, *J* = 1.8 Hz, Ar-H).
**MS-ESI** *m*/*z* (rel. int.): 267.1 ([MH]⁺, 35), 182.0 (100).
**HPLC:** Method A, detection UV 254 nm, RT = 3.7 min, peak area 98.0 %.

### 4-(5-(2-Methoxy-6-nitro-4-((piperidin-1-yl)methyl)phenoxy)pentylthio)-7-(trifluoromethyl)quinoline 16.

2-Methoxy-6-nitro-4-((piperidin-1-yl)methyl)phenol (352 mg, 1.30 mmol), Cs₂CO₃ (425 mg, 1.30 mmol), NaI (85 mg, 1.30 mmol) and anhydrous DMF (5 mL) were charged in a 25 ml round-bottomed flask equipped with a magnetic stirrer under inert atmosphere. 4-(5-Bromopentylthio)-7-(trifluoromethyl)quinoline (500 mg, 1.30 mmol) was added and the reaction mixture was stirred for 3 h at 90 °C. After evaporation of the DMF, the reaction mixture was poured in 50 mL of H₂O and extracted with EtOAc (2 x 100 mL). The organic layer was washed with brine (2 x 20 mL), dried over MgSO₄, filtered and evaporated to dryness. The crude compound was purified by column chromatography (SiO₂; EtOAc:MeOH = 94:6) to give after evaporation a pure solid. The compound was dried under vacuum to yield to 4-(5-(2-methoxy-6-nitro-4-((piperidin-1-yl)methyl)phenoxy)pentylthio)-7-(trifluoromethyl)quinoline **16** (240 mg, 33 % yield) as a viscous brown oil. 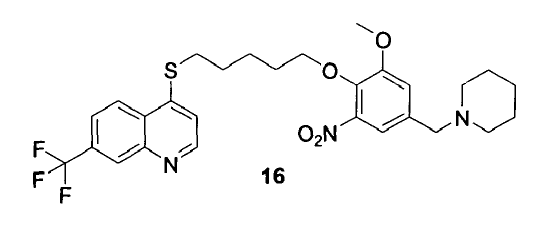 **MW:** 563.63; **Yield:** 60 %; Viscous Brown Oil.
***R_{f}*:** 0.30 (EtOAc:MeOH = 9:1).
**¹H-NMR** (CDCl₃, δ): 1.40-1.50 (m, 2H, CH₂), 1.52-1.62 (m, 4H, 2CH₂), 1.68-1.80 (m, 2H, CH₂), 1.80-1.96 (m, 4H, 2CH₂), 2.36 (s broad, 4H, NCH₂), 3.16 (t, 2H, *J* = 7.3 Hz, S-CH₂), 3.41 (s, 2H, N-CH₂), 3.89 (s, 3H, O-CH₃), 4.15 (t, 2H, *J* = 6.1 Hz, O-CH₂), 4.18 (s, 2H, N-CH₂), 7.13 (d, 1H, *J* = 1.8 Hz, Ar-H), 7.25-7.32 (m, 2H, Ar-H) 7.70 (dd, 1H, *J* = 8.8 Hz, *J* = 1.8 Hz, Ar-H), 8.23 (d, 1H, *J* = 8.8 Hz, Ar-H), 8.35 (s, 1H, Ar-H), 8.78 (d, 1H, *J* = 4.8 Hz, Ar-H).
**MS-ESI** *m*/*z* (rel. int.): 564.1 ([MH]⁺, 65), 182.1 (100).
**HPLC:** Method A, detection UV 254 nm, RT = 5.2 min, peak area 96.5 %.

### Example 17:

### Inhibition of Aβ production in vitro

An exemplary assay for the analysis of inhibition of beta-secretase activity utilizes the human embryonic kidney cell line HEK-293 (ATCC Accession No. CRL-1573) stably transfected with APP751 containing the naturally occurring double mutation Lys651Met52 to Asn651Leu652 (numbered for APP751), commonly called the "Swedish mutation" and shown to overproduce Aβ 40 and Aβ 42 (Citron et.al., 1992, Nature 360:672-674). The cells were plated in 10 cm plates and in Dulbecco's Modified Eagle's medium (DMEM, Sigma D-6546) containing 10% fetal bovine serum. After cells are established (3 days post plating), cells are incubated in the presence/absence of the inhibitory compound (diluted in DMSO) at the desired concentration, generally from 0.2 to 20µM, and with a final concentration of DMSO of 0.1 %. Cells were incubated for 7 hours in the presence of phosphoramidon (1 µM) (Sigma). Media and cell lysates were collected, centrifuged, normalized to total protein and assayed for Aβ 40 and Aβ 42 by sandwich ELISA according to the manufacturer's instructions (Biosource International). For Aβ 42 detection, samples are concentrated on YM3 Microcon columns (Millipore). Using specific antibodies to detect cleavage product, for example, Aβ 40, the enzymatic activity is measured in the presence and absence of the compound inhibitors to demonstrate specific inhibition of APP cleavage. Aβ levels are determined by the difference between the Aβ values for the compound treatment and baseline periods. Dose response curves are plotted as percent inhibition versus compound concentration.
As shown in Table 1, compounds of the invention demonstrate reduced Aβ 40 levels in conditioned medium, indicative of an inhibitory effect on APP processing and Aβ production.

**Table 1. IC50 of compounds of the invention for the inhibition of Aβ 40 production in HEK-293 cells overexpressing swAPP751**

| | IC50 (µM) |
|---|---|
| Compound 1 | 11.6 |
| Compound 2 | 2.4 |
| Compound 3 | 1.9 |
| Compound 5 | 5.16 |
| Compound 8 | 5.13 |
| Compound 10 | 8.5 |
| Compound 11 | 2.45 |
| Compound 12 | 7.3 |
| Compound 13 | 8.26 |
| Compound 14 | 0.72 |

### Inhibition of Aβ in Animal Models of AD

Various animal models can be used to screen for inhibition of beta-secretase activity. Examples of animal models useful in the invention include, but are not limited to, mouse, guinea pig, and the like. The animals used can be wild type, transgenic, or knockout models.
Animals are administered an amount of the compound inhibitor formulated appropriately in PBS. Control animals are untreated, or treated with an inactive compound. Administration is repeated daily for a period of days. Beginning on day 0, brain tissue or fluid is obtained from selected animals and analyzed for the presence of APP cleavage peptides, including beta, using the specific antibodies to Aβ. At the end of the test period, animals are sacrificed and brain tissue or fluid is analyzed for the presence of Aβ and/or β amyloid plaques. The tissue is also analyzed for necrosis.

Animals can be Male Hartley albino guinea-pigs, weighing 250 - 270 g at delivery, obtained from Charles River Laboratories (L'Arbresle, France) and are treated once a day for 15 consecutive days and by the i.p. route. One hour after the final administration, the guinea-pigs are killed and brains are immediately extracted and immersed in an oxygenated (95 % O₂ / 5 % CO₂) physiological saline bath placed on ice (1-2 °C) and superficial vessels were removed. The whole brains is dissected to provide left and right cortices, which are weighted, snap frozen in liquid nitrogen, and stored at -80 °C separately. The maximum time between sacrifice and snap freezing is less than 15 minutes. Right cortices are homogenized for 3 h at room temperature in 5 M Guanidine-Hcl, 50mM Tris-Hcl, pH 8 with a protease inhibitor mixture (Roche Diagnostics). Tissue homogenates are diluted 1:1 (v/v) in BSAT-DPBS buffer (Dulbecco's phosphate buffered saline with 5 % BSA and 0.03 % Tween-20), pH 7.4, and are centrifuged at 20,000 g for 20min at 4°C. Supernatants were diluted 1:1 (v/v) in ELISA kit sample buffer, normalized to total protein and assayed for Aβ 40 and Aβ 42 by sandwich ELISA according to the manufacturer's instructions. For Aβ 42 detection, samples are concentrated on YM3 Microcon columns (Millipore). The protocol ensures a final concentration of guanidine inferior to 0.1 M, as recommended by the manufacturer and ELISA standards included 0.1 M guanidine.

Animals administered with the compound inhibitors are expected to demonstrate reduced Aβ in brain tissues and fluids, and reduced beta amyloid plaques in brain tissue, as compared with non-treated controls.

### Inhibition of Aβ Production in Human Patients

Patients suffering from Alzheimer's Disease (AD) demonstrate an increased amount of Aβ in the brain. AD patients, patients at risk for developing AD identified either by recognition of a familial inheritance pattern, for example, presence of the Swedish Mutation, and/or by monitoring diagnostic parameters, are administered an amount of the compound inhibitor diluted in PBS. Administration is repeated daily for the duration of the test period. Beginning on day 0, cognitive and memory tests are performed once per week.

Patients administered with the compound inhibitors are expected to demonstrate cognitive and memory scores are expected to slow and/or stabilize as compared with non-treated patients.

## Claims

1. A compound having a general formula (I): 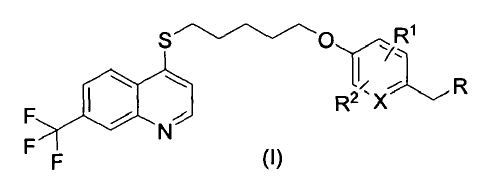 wherein:
X is CH or N;
R¹ and R², which are the same or different, represent an hydrogen atom, a halogen atom, C₁-C₆ alkyl, C₁-C₆ alkoxy group, hydroxyl group, NO₂, or NR'R", wherein R' and R", which are the same or different, represent an hydrogen atom or a C₁-C₆ alkyl group;
R is an hydrogen atom, hydroxyl, 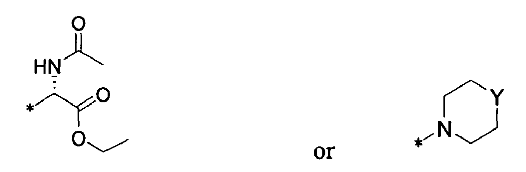 wherein Y represents an oxygen atom, a CH₂ radical or NR³, in which R³ represents an hydrogen atom, -(C=O)C(CH₃)₃, -(C=O)N(C₂H₅)₂ or -(C=O)N[CH(CH₃)₂]₂;
a salt, hydrate or mixture therof.

2. The compound according to claim 1, wherein X = CH and R is OH,

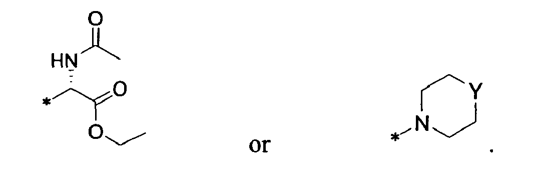

3. The compound according to claim 1 or 2, wherein X = CH and R is OH or and R¹ and R² both represent an hydrogen atom.

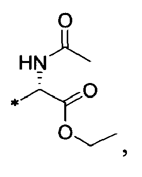

4. The compound according to claim 1 or 2, wherein X = CH and R is 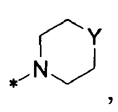 Y is CH₂ and R¹ and R² are (i) both hydrogen atoms or (ii) one is hydrogen and the other is alkyl, preferably methyl (even more preferably 2-Me), halogen atom, preferably chlorine (even more preferably 2-Cl), nitro (preferably 2-NO₂), NR'R" as defined above, preferably NH₂ (even more preferably 2-NH₂), alkoxy, preferably methoxy (even more preferably 2-OMe), or (iii) both are different from hydrogen, in particular one is alkoxy, preferably methoxy (even more preferably 2-OMe) and the other is nitro (preferably 5-NO₂).

5. The compound according to claim 1 or 2, wherein X = CH and R is 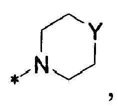 Y is oxygen atom and R¹ and R² are (i) both hydrogen atoms or (ii) one is hydrogen and the other is alkoxy, preferably methoxy (even more preferably 2-OMe).

6. The compound according to claim 1 or 2, wherein X = CH and R is 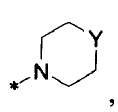 Y is NR³, with R³ as defined in claim 1, and R¹ and R² both represent H.

7. The compound according to claim 1, wherein X = N and R, R¹ and R² are H.

8. The compound according to claim 1, selected in the group consisting of:
4-(5-(4-((piperidin-1-yl)methyl)phenoxy)pentylthio)-7-(trifluoromethyl)quinoline dihydrochloride,
4-(5-(2-methoxy-4-((piperidin-1-yl)methyl)phenoxy)pentylthio)-7-(trifluoromethyl)quinoline dihydrochloride,
4-(5-(4-(morpholinomethyl)phenoxy)pentylthio)-7-(trifluoromethyl)quinoline dihydrochloride,
4-(5-(2-methoxy-4-(morpholinomethyl)phenoxy)pentylthio)-7-(trifluoromethyl)quinoline dihydrochloride,
4-(5-(4-((piperazin-1-yl)methyl)phenoxy)pentylthio)-7-(trifluoromethyl)quinoline trihydrochloride,
4-(5-(2-chloro-4-((piperidin-1-yl)methyl)phenoxy)pentylthio)-7-(trifluoromethyl)quinoline dihydrochloride,
4-(5-(2-nitro-4-((piperidin-1-yl)methyl)phenoxy)pentylthio)-7-(trifluoromethyl)quinoline dihydrochloride,
4-(4-(5-(7-(trifluoromethyl)quinolin-4-ylthio)pentyloxy)benzyl)-*N,N*-diethylpiperazine-1-carboxamide dihydrochloride,
1-(4-(4-(5-(7-(trifluoromethyl)quinolin-4-ylthio)pentyloxy)benzyl)piperazin-1-yl)-2,2-dimethylpropan-1-one dihydrochloride,
4-(4-(5-(7-(trifluoromethyl)quinolin-4-ylthio)pentyloxy)benzyl)-*N,N* diisopropylpiperazine-1-carboxamide dihydrochloride,
(4-(5-(7-(trifluoromethyl)quinolin-4-ylthio)pentyloxy)phenyl)methanol hydrochloride,
2-(5-(7-(trifluoromethyl)quinolin-4-ylthio)pentyloxy)-5-((piperidin-1-yl)methyl)benzenamine trihydrochloride,
4-(5-(2-methyl-4-((piperidin-1-yl)methyl)phenoxy)pentylthio)-7-(trifluoromethyl)quinoline dihydrochloride,
(*S*)-ethyl 3-(4-(5-(7-(trifluoromethyl)quinolin-4-ylthio)pentyloxy)phenyl)-2-acetamidopropanoate hydrochloride,
4-(5-(6-methylpyridin-3-yloxy)pentylthio)-7-(trifluoromethyl)quinoline dihydrochloride and 4-(5-(2-Methoxy-6-nitro-4-((piperidin-1-yl)methyl)phenoxy)pentylthio)-7-(trifluoromethyl)quinoline.

9. A pharmaceutical composition comprising at least one compound as defined in any one of the preceding claims, and a pharmaceutically acceptable support, optionally in association with another active agent.

10. The pharmaceutical composition according to claim 9, for preventing or treating a Amyloid-β-Peptide Related Disorder.

11. The pharmaceutical composition according to claim 9, for preventing or treating Alzheimer's disease.

12. The pharmaceutical composition according to claim 9, for preventing or treating mild cognitive impairment, Down's syndrome, Hereditary Cerebral Hemorrhage with Amyloidosis of the Dutch-Type, cerebral amyloid angiopathy and consequences thereof, i.e. single and recurrent lobar hemorrhages, other degenerative dementias, including dementias of mixed vascular and degenerative origin, dementia associated with Parkinson's disease, dementia associated with progressive supranuclear palsy, dementia associated with cortical basal degeneration, or diffuse Lewy body type of Alzheimer's disease.

13. The pharmaceutical composition according to claim 9, for preventing disease or treating patients with MCI (mild cognitive impairment) and preventing or delaying the onset of Alzheimer's disease in those who would progress from MCI to AD, for treating or preventing Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, dementia associated with progressive supranuclear palsy, dementia associated with cortical basal degeneration, or diffuse Lewy body type of Alzheimer's disease.
